# EUROPÄISCHE PATENTSCHRIFT

(11) **EP 1 387 838 B1**
(45) Veröffentlichungstag und Bekanntmachung des Hinweises auf die Patenterteilung: **19.04.2006**
(21) Anmeldenummer: 02748691.9
(22) Anmeldetag: 03.05.2002
(51) Int. Cl.: C07D 401/12, C07D 401/14, C07D 405/12, A61K 31/44, A61P 19/02, A61P 33/00

(54) **CYANOANTHRANYLAMID-DERIVATE UND DEREN VERWENDUNG ALS ARZNEIMITTEL**
CYANOANTHRANILAMIDE DERIVATIVES AND THE USE THEREOF AS MEDICAMENTS
DERIVES DE CYANOANTHRANYLAMIDE ET LEUR UTILISATION COMME MEDICAMENT

(30) Priorität: 08.05.2001 DE 10123587; 15.05.2001 DE 10125295
(43) Veröffentlichungstag der Anmeldung: 11.02.2004
(73) Patentinhaber: Schering Aktiengesellschaft, 13342 Berlin (DE)
(72) Erfinder: ERNST, Alexander, CH-4052 Basel (CH); HUTH, Andreas, 12437 Berlin (DE); KRÜGER, Martin, 13465 Berlin (DE); THIERAUCH, Karl-Heinz, 14169 Berlin (DE); MENRAD, Andreas, 16515 Oranienburg (DE); HABEREY, Martin, 12169 Berlin (DE)
(86) Internationale Anmeldenummer: PCT/EP2002/004921
(87) Internationale Veröffentlichungsnummer: WO 2003/000678

(56) Entgegenhaltungen:
- WO-A-00/27819
- WO-A-01/85671
- WO-A-02/055501
- WERMUTH ET AL: "The Practise of Medicinal Chemistry" PRACTICE OF MEDICINAL CHEMISTRY, XX, XX, 1996, Seiten 203-237, XP002190259

## Beschreibung

Die Erfindung betrifft substituierte Cyanoanthranylamid-Derivate, deren Herstellung und Verwendung als Arzneimittel zur Behandlung von Erkrankungen, die durch persistente Angiogenese ausgelöst werden.

Persistente Angiogenese kann die Ursache für verschiedene Erkrankungen wie Psoriasis, Arthritis, wie rheumatöide Arthritis, Hämangioma, Angiofribroma, Augenerkrankungen, wie diabetische Retinopathie, Neovaskulares Glaukom, Nierenerkrankungen, wie Glomerulonephritis, diabetische Nephropathie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen und Artheriosklerose sein oder zu einer Verschlimmerung dieser Erkrankungen führen.

Die persistente Angiogenese wird durch den Faktor VEGF über seinen Rezeptor induziert. Damit VEGF diese Wirkung entfalten kann ist es nötig, daß VEGF am Rezeptor bindet und eine Tyrosinphosphorylierung hervorgerufen wird.

Eine direkte oder indirekte Inhibition des VEGF-Rezeptors (VEGF = vaskulärer endothelialer Wachstumsfaktor) kann zur Behandlung derartiger Erkrankungen und anderer VEGF-induzierter pathologischer Angiogenese und vaskularer permeabiler Bedingungen, wie Tumor-Vaskularisierung, verwendet werden. Beispielsweise ist bekannt, daß durch lösliche Rezeptoren und Antikörper gegen VEGF das Wachstum von Tumoren gehemmt werden kann.

Aus der WO 00/27819 sind Anthranylsäureamide bekannt, die als Arzneimittel zur Behandlung von Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, zum Einsatz kommen.

Die bekannten Verbindungen sind in den angegebenen Indikationen zwar allgemein wirksam, aber ihre Wirksamkeit geht in der Regel einher mit einem inhibitorischen Potential gegenüber metabolisierenden Enzymen der Leber (Cytochrom P450 Isoenzyme). Dies birgt die Gefahr von unerwünschten Arzneimittelwechselwirkungen und dadurch bedingt eine schlechtere Verträglichkeit des Medikaments.

Es besteht daher ein Wunsch nach einerseits wirksameren und andererseits toxikologisch unbedenklicheren Verbindungen, die darüberhinaus auch noch besser verträglich sein sollten.

Es wurde nun gefunden, daß Verbindungen der allgemeinen Formel I 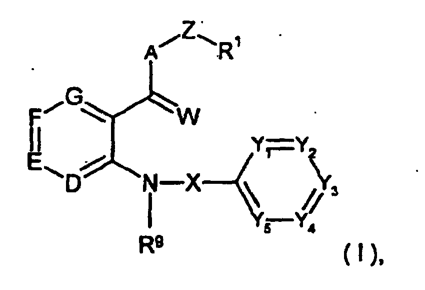 in der
- A: für die Gruppe -N(R⁷)- steht.
- W: für Sauerstoff, Schwefel, zwei Wasserstoffatome oder die Gruppe -N(R⁸)- steht,
- Z: für eine Bindung, die Gruppe -N(R¹⁰)- oder =N-, für verzweigtes oder unverzweigtes C₁-C₁₂-Alkyl oder für die Gruppe 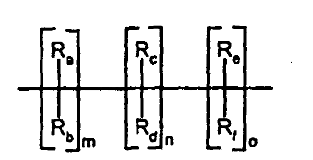 steht,
- m, n und o: für 0 - 3 stehen,
- Rₐ, R_{b}, R_{c}, R_{d}, Rₑ, R_{f}: unabhängig voneinander für Wasserstoff, Fluor, C₁-C₄ Alkyl oder die Gruppe -N(R¹¹)- stehen und/ oder Rₐ und/ oder R_{b} mit R_{c} und/ oder R_{d} oder R_{c} mit Rₑ und/ oder R_{f} eine Bindung bilden können, oder bis zu zwei der Reste Rₐ-R_{f} eine Brücke mit je bis zu 3 C-Atomen zu R¹ oder zu R⁷ schließen können,
- X: für C₁-C₆-Alkyl steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl und/ oder mit der Gruppe-NR¹²R¹³ substituiertes verzweigtes oder unverzweigtes C₁-C₁₂-Alkyl oder C₂-C₁₂-Alkenyl steht; oder für gegebenenfalls ein- oder mehrfach, gleich oder verschieden mit Halogen, Hydroxy, C₁-C₆-Alkyloxy, C₁-C₆₋Alkyl und/ oder mit der Gruppe -NR¹²R¹³ substituiertes C₃₋C₁₀-Cycloalkyl oder C₃-C₁₀-Cycloalkenyl steht; oder für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl, Cyano-C₁-C₆₋alkyl oder mit der Gruppe =O, OR¹⁴ oder R¹⁴ substituiertes Aryl oder Hetaryl steht,
- Y₁ bis Y₅: jeweils für ein Stickstoffatom oder für die Gruppe -CY⁶ stehen,
- Y⁶: für Cyano, Halogen, C₁-C₆-Alkyl, C₁-C₆-Alkoxy, Amino oder Hydroxy steht, wobei im Ring mindestens ein Stickstoff-Atom enthalten ist und am Ring mindestens ein Cyano-Rest enthalten ist,
- D: für ein Stickstoffatom oder für die Gruppe C-R³ steht,
- E: für ein Stickstoffatom oder für die Gruppe C-R⁴ steht,
- F: für ein Stickstoffatom oder für die Gruppe C-R⁵ steht,
- G: für ein Stickstoffatom oder für die Gruppe C-R⁶ steht, wobei
- R³, R⁴ ,R⁵ und R⁶: für Wasserstoff, Halogen oder unsubstituiertes oder gegebenenfalls ein- oder mehrfach mit Halogen substituiertes C₁-C₆-Alkoxy, C₁-C₆-Alkyl oder C₁-C₆₋Carboxyalkyl stehen,
- R⁷: für Wasserstoff oder C₁-C₆-Alkyl steht oder mit Rₐ-R_{f} von Z oder zu R¹ eine Brücke, mit bis zu 3 Ringgliedem bildet,
- R⁸, R⁹ R¹⁰ und R¹¹: für Wasserstoff oder C₁-C₆-Alkyl stehen,
- R¹² und R¹³: für Wasserstoff, C₁-C₆-Alkyl stehen oder einen Ring bilden,der ein weiteres Heteroatom enthalten kann,
- R¹⁴: für die Gruppe C₁-C₁₅-Alkyl-R¹⁵, in der der Alkylrest ein- oder mehrfach durch Sauerstoff unterbrochen sein kann, oder für die Gruppe (CH₂-CH₂-O)ᵤ(CH₂)ᵥ-R¹⁵ steht,
- R¹⁵: für Aryl, Hetaryl, C₁-C₆-Alkyl, Aralkyl, -CH₂CN oder für die Gruppe NR¹⁶R¹⁷ steht,
- R¹⁶ und R¹⁷: für Wasserstoff, C₁-C₆-Alkyl, C₁-C₆-Acyl stehen oder einen Ring bilden,der ein weiteres Heteroatom enthalten kann, und
- u und v: für 0-5 stehen, bedeuten, sowie deren Isomeren, Enantiomeren und Salze, die oben aufgeführten Nachteile überwinden.

Die erfindungsgemäßen Verbindungen verhindern eine Tyrosinphosphorylierung bzw. stoppen die persistente Angiogenese und damit das Wachstum und ein Ausbreiten von Tumoren, wobei sie sich insbesondere durch eine geringere Inhibition von Isoformen des Cytochroms P 450 (2C9 und 2C19) auszeichnen. Die Medikation mit den erfindungsgemäßen Verbindungen kann daher auch ohne Rücksicht auf begleitend verabreichte Arzneimittel, die über diese Isoformen abgebaut werden, risikolos erfolgen.

Falls R⁷ eine Brücke zu R¹ bildet, entstehen Heterocyclen, an die R¹ ankondensiert ist. Beispielsweise seien genannt:

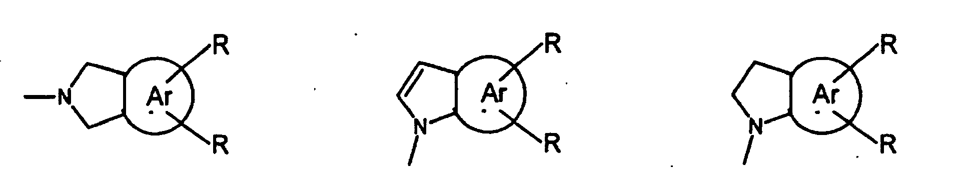

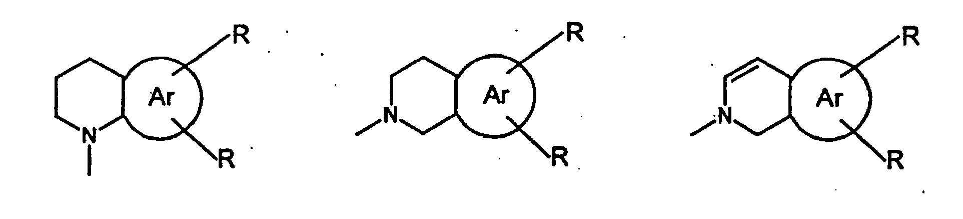

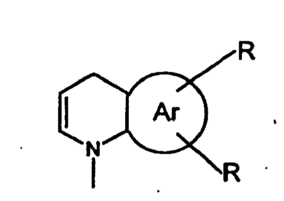

Stellen Rₐ, R_{b}, R_{c}, R_{d}, Rₑ, R_{f} unabhängig voneinander Wasserstoff oder C₁-C₄ Alkyl dar, so bildet Z eine Alkylkette.
Bilden Rₐ und/ oder R_{b} mit R_{c} und/ oder R_{d} oder R_{c} und/ oder R_{d} mit Rₑ und/ oder R_{f} eine Bindung, so steht Z für eine Alkenyl- oder Alkinylkette.

Bilden Rₐ - R_{f} eine Brücke mit sich selbst, so stellt Z eine Cycloalkyl- oder Cycloalkenylgruppe dar.

Bilden bis zu zwei der Reste Rₐ-R_{f} eine Brücke mit bis zu 3 C-Atomen zu R¹ aus, so ist Z zusammen mit R¹ ein benzo- oder hetarylkondensiertes (Ar) Cycloalkyl. Beispielsweise seien genannt:

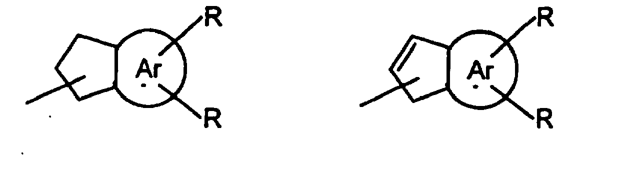

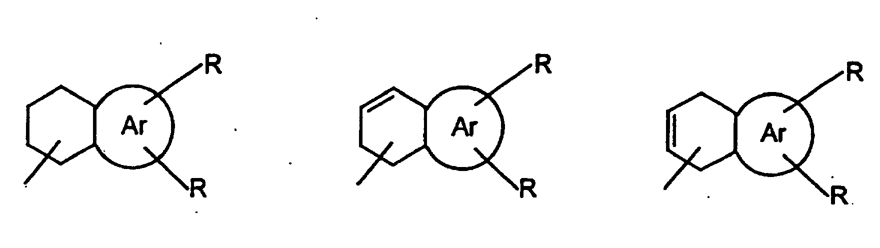

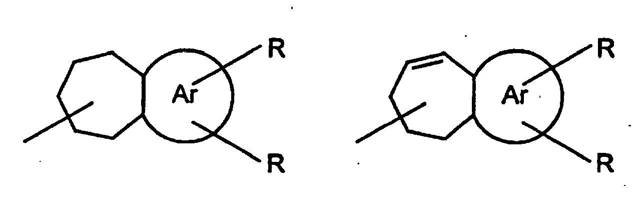

Schließt einer der Reste Rₐ - R_{f} zu R⁷ eine Brücke, so bildet sich ein Stickstoffheterozyklus, der durch eine Gruppe von R¹ getrennt sein kann. Beispielsweise seien genannt:

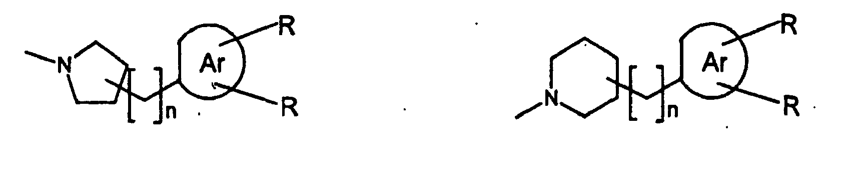

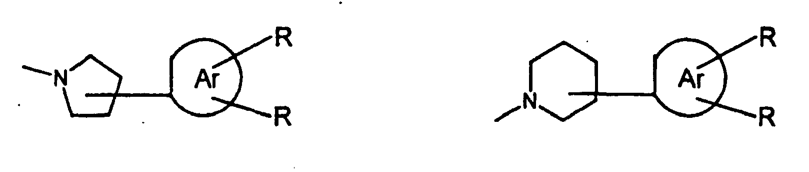

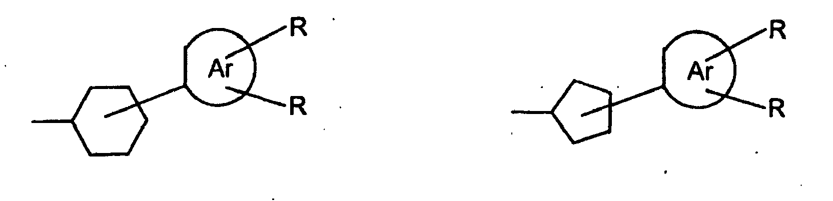

Unter Alkyl ist jeweils ein geradkettiger oder verzweigter Alkylrest, wie beispielsweise Methyl, Ethyl, Propyl, Isopropyl, Butyl, Isobutyl, sek. Butyl, Pentyl, Isopentyl oder Hexyl, Heptyl, Octyl, Nonyl; Decyl, Undecyl, Dodecyl zu verstehen.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, Pentyloxy, Isopentyloxy, yHexyloxy, Heptyloxy, Octyloxy, Nonyloxy, Decyloxy, Undecyloxy oder Dodecyloxy zu verstehen.

Unter Cycloalkyl sind monocyclische Alkylringe wie Cyclopropyl, Cyclobutyl, Cyclopentyl, Cyclohexyl oder Cycloheptyl, Cyclooctyl, Cyclononyl oder Cyclodecyl, aber auch bicyclische Ringe oder tricyclische Ringe wie zum Beispiel Adamantanyl zu verstehen.

Unter Cycloalkenyl ist jeweils Cyclobutenyl, Cyclopentenyl, Cyclohexenyl, Cycloheptenyl, Cyclooctenyl, Cyclononenyl oder Cyclodecenyl zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Unter Alkenyl ist jeweils ein geradkettiger oder verzweigter Alkenyl-Rest zu verstehen, der 2 - 6, bevorzugt 2 - 4 C-Atome enthält. Beispielsweise seien die folgenden Reste genannt: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, But-3-en-1-yl, Allyl.

Der Arylrest hat jeweils 6 - 12 Kohlenstoffatome wie beispielsweise Naphthyl, Biphenyl und insbesondere Phenyl.

Der Heteroarylrest umfaßt jeweils 3-16 Ringatome und kann anstelle des Kohlenstoffs ein- oder mehrere, gleiche oder verschiedene Heteroatome, wie Sauerstoff, Stickstoff oder Schwefel im Ring enthalten, und kann mono-, bi- oder tricyclisch sein, und kann zusätzlich jeweils benzokondensiert sein.

### Beispielsweise seien genannt:

Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, etc. und Benzoderivate davon, wie z. B. Benzofuranyl, Benzothienyl, Benzoxazolyl, Benzimidazolyl, Indazolyl, Indolyl, Isoindolyl, etc.; oder Pyridyl, Pyridazinyl, Pyrimidinyl, Pyrazinyl, Triazinyl, etc. und Benzoderivate davon, wie z. B. Chinolyl, Isochinolyi, etc.;
Azocinyl, Indolizinyl, Benzimidazolyl, Purinyl, etc. und Benzoderivate davon; Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl oder Oxepinyl,etc.

Der Aryl- und der Heteroarylrest kann jeweils 1-, 2- oder 3-fach gleich oder verschieden substitutiert sein mit Hydroxy, Halogen, C₁-C₄-Alkoxy, mit C₁-C₄-Alkyl, ein oder mehrfach mit Halogen substituiertes C₁-C₄-Alkyl.

Ist eine saure Funktion enthalten, sind als Salze die physiologisch verträglichen Salze organischer und anorganischer Basen geeignet wie beispielsweise die gut löslichen Alkali- und Erdalkalisalze sowie N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten sind die physiologisch verträglichen Salze organischer und anorganischer Säuren geeignet wie Salzsäure, Schwefelsäure, Phosphorsäure, Zitronensäure, Weinsäure, Fumarsäue u.a.

Die erfindungsgemäßen Verbindungen der allgemeinen Formel beinhalten auch die möglichen tautomeren Formen und umfassen die E- oder Z-Isomeren oder, falls ein chirales Zentrum vorhanden ist, auch die Racemate und Enantiomeren.

Als besonders wirksam haben sich solche Verbindungen der allgemeinen Formel I erwiesen, in der
- A: für die Gruppe -N(R⁷)- steht,
- W: für Sauerstoff steht,
- Z: für eine Bindung oder für verzweigtes oder unverzweigtes C₁-C₁₂-Alkyl steht,
- X: für C₁-C₆-Alkyl steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl und/ oder mit der Gruppe-NR¹²R¹³ substituiertes verzweigtes oder unverzweigtes C₁-C₁₂-Alkyl oder C₂C₁₂-Alkenyl steht; oder für gegebenenfalls ein- oder mehrfach, gleich oder verschieden mit Halogen, Hydroxy, C₁-C₆-Alkyloxy, C₁-C₆₋Alkyl und/ oder mit der Gruppe -NR¹²R¹³ substituiertes C₃-C₁₀-Cycloalkyl oder C₃-C₁₀₋Cycloalkenyl steht; oder für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl, Cyano-C₁-C₆-alkyl oder mit der Gruppe =O, OR¹⁴ oder R¹⁴ substituiertes Aryl oder Hetaryl steht,
- Y₁ bis Y₅: jeweils für ein Stickstoffatom oder für die Gruppe -CY⁶ stehen,
- Y⁶: für Cyano, Halogen, C₁-C₆-Alkyl, C₁-C₆-Alkoxy, Amino oder Hydroxy steht, wobei im Ring mindestens ein Stickstoff-Atom enthalten ist und am Ring mindestens ein Cyano-Rest enthalten ist,
- D: für ein Stickstoffatom oder für die Gruppe C-R³ steht,
- E: für ein Stickstoffatom oder für die Gruppe C-R⁴ steht,
- F: für ein Stickstoffatom oder für die Gruppe C-R⁵ steht,
- G: für ein Stickstoffatom oder für die Gruppe C-R⁶ steht, wobei
- R³, R⁴, R⁵ und R⁶: für Wasserstoff, Halogen oder unsubstituiertes oder gegebenenfalls ein- oder mehrfach mit Halogen substituiertes C₁-C₆-Alkoxy, C₁-C₆-Alkyl oder C₁-C₆₋Carboxyalkyl stehen,
- R⁷: für Wasserstoff oder C₁-C₆-Alkyl steht oder mit Rₐ-R_{f} von Z oder zu R¹ eine Brücke mit bis zu 3 Ringgliedem bildet,
- R⁹: für Wasserstoff oder C₁-C₆-Alkyl steht,
- R¹² und R¹³: für Wasserstoff, C₁-C₆-Alkyl stehen oder einen Ring bilden,der ein weiteres Heteroatom enthalten kann,
- R¹⁴: für die Gruppe (CH₂-CH₂-O)ᵤ(CH₂)ᵥ-R¹⁵ steht,
- R¹⁵: für Aryl, Hetaryl, C₁₋₆-Alkyl, Aralkyl, -CH₂CN oder für die Gruppe NR¹⁶R¹⁷ steht,
- R¹⁶ und R¹⁷: für Wasserstoff, C₁-C₆-Alkyl, C₁-C₆-Acyl stehen oder einen Ring bilden,der ein weiteres Heteroatom enthalten kann, und
- u und v: für 0-5 stehen, bedeuten, sowie deren Isomeren, Enantiomeren und Salze.

Insbesondere wirksam sind solche Verbindungen der allgemeinen Formel I, in der
- A: für die Gruppe -N(R⁷)- steht,
- W: für Sauerstoff steht,
- Z: für eine Bindung oder für verzweigtes oder unverzweigtes C₁-C₁₂-Alkyl steht,
- X: für C₁-C₆-Alkyl steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl, Cyano-C₁-C₆₋alkyl, oder mit der Gruppe =O, OR¹⁴ oder R¹⁴ substituiertes Aryl oder Hetaryl steht,
- Y₁ bis Y₅: gemeinsam für einen Pyridyl-Ring stehen, der mit einer Cyanogruppe substituiert ist und der zusätzlich mit Halogen, C₁-C₆-Alkyl, C₁-C₆-Alkoxy, Amino, C₁-C₆₋Carboxyalkylamino oder Hydroxy substituiert sein kann,
- D: für ein Stickstoffatom oder für die Gruppe C-R³ steht,
- E: für ein Stickstoffatom oder für die Gruppe C-R⁴ steht,
- F: für ein Stickstoffatom oder für die Gruppe C-R⁵ steht,
- G: für ein Stickstoffatom oder für die Gruppe C-R⁶ steht, wobei
- R³,R⁴ ,R⁵ und R⁶: für Wasserstoff, Halogen oder unsubstituiertes oder gegebenenfalls ein- oder mehrfach mit Halogen substituiertes C₁-C₆-Alkoxy, C₁-C₆-Alkyl oder C₁-C₆₋Carboxyalkyl stehen,
- R⁷: für Wasserstoff oder C₁-C₆-Alkyl steht oder mit Rₐ-R_{f} von Z oder zu R¹ eine Brücke mit bis zu 3 Ringgliedem bildet,
- R⁹: für Wasserstoff oder C₁-C₆-Alkyl steht,
- R¹⁴: für die Gruppe (CH₂-CH₂-O)ᵤ(CH₂)ᵥ-R¹⁵ steht,
- R¹⁵: für Aryl, Hetaryl, C₁₋₆-Alkyl, Aralkyl, -CH₂CN oder für die Gruppe NR¹⁶R¹⁷ steht,
- R¹⁶ und R¹⁷: für Wasserstoff, C₁-C₆-Alkyl, C₁-C₆-Acyl stehen oder einen Ring bilden,der ein weiteres Heteroatom enthalten kann, und
- u und v: für 0-5 stehen, bedeuten, sowie deren Isomeren, Enatiomeren und Salze.

Ganz besonders wirksam sind solche Verbindungen der allgemeinen Formel I, in der
- A: für die Gruppe -N(R⁷)- steht,
- W: für Sauerstoff steht,
- Z: für eine Bindung oder C₁-C₆-Alkyl steht,
- X: für C₁-C₆-Alkyl steht,
- R¹: für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl, Cyano-C₁-C₆₋alkyl oder mit der Gruppe =O substituiertes Thiophen, Furan, Oxazol, Thiazol, Indazolyl, Imidazol, Pyrazol, Pyridin, Pyrimidin, Triazin, Chinolin, Isochinolin oder 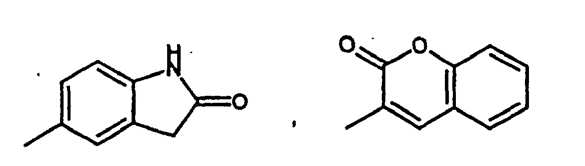 oder 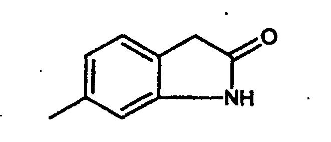 steht,
- Y₁ bis Y₅: gemeinsam für einen Pyridyl-Ring stehen, der mit einer Cyanogruppe substituiert ist und der zusätzlich mit Halogen substituiert sein kann,
- D: für ein Stickstoffatom oder für die Gruppe C-R³ steht,
- E: für die Gruppe C-R⁴ steht,
- F: für die Gruppe C-R⁵ steht,
- G: für die Gruppe C-R⁶ steht, wobei
- R³, R⁴, R⁵ und R⁶: für Wasserstoff stehen,
- R⁷: für Wasserstoff steht und
- R⁹: für Wasserstoff steht, bedeuten, sowie deren Isomeren,
Enantiomeren und Salze.

Ebenfalls ganz besonders wirksam sind solche Verbindungen der allgemeinen Formel I, in der
- A: für die Gruppe -N(R⁷)- steht,
- W: für Sauerstoff steht,
- Z: für eine Bindung steht,
- X: für C₁-C₆-Alkyl steht,
- R¹: für gegebenenfalls ein- oder mehrfach, gleich oder verschieden mit C₁-C₆-Alkyloxy, C₁-C₆-Alkyl, Cyano-C₁-C₆₋alkyl oder mit der Gruppe =O substituiertes Indazolyl, Chinolin, Isochinolin oder 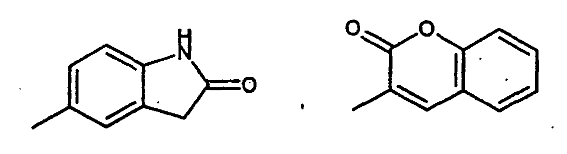 oder 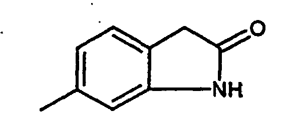 steht,
- Y₁ bis Y₅: gemeinsam für einen Pyridyl-Ring stehen, der mit einer Cyanogruppe substituiert ist und der zusätzlich mit Halogen substituiert sein kann,
- D: für ein Stickstoffatom oder für die Gruppe C-R³ steht,
- E: für die Gruppe C-R⁴ steht,
- F: für die Gruppe C-R⁵ steht,
- G: für die Gruppe C-R⁶ steht, wobei
- R³, R⁴, R⁵ und R⁶: für Wasserstoff stehen,
- R⁷: für Wasserstoff steht und
- R⁹: für Wasserstoff steht, bedeuten, sowie deren Isomeren,
Enantiomeren und Salze.

Die Verbindungen der Formel I sowie deren physiologisch verträglichen Salze sind auf Grund ihrer inhibitorischen Aktivität in Bezug auf Phosphorylierung des VEGF-Rezeptors als Arzneimittel verwendbar. Auf Grund ihres Wirkprofils eignen sich die erfindungsgemäßen Verbindungen zur Behandlung von Erkrankungen, die durch eine persistente Angiogenese hervorgerufen oder gefördert werden.

Da die Verbindungen der Formel I als Inhibitoren der Tyrosinkinase KDR und FLT identifiziert werden, eignen sie sich insbesondere zur Behandlung von solchen Krankheiten, die durch die über den VEGF-Rezeptor ausgelöste persistente Angiogenese oder eine Erhöhung der Gefäßpermeabilität hervorgerufen oder gefördert werden.

Gegenstand der vorliegenden Erfindung ist auch die Verwendung der erfindungsgemäßen Verbindungen als Inhibitoren der Tyrosinkinase KDR und FLT.

Gegenstand der vorliegenden Erfindung sind somit auch Arzneimittel zur Behandlung von Tumoren bzw. deren Verwendung.

Die erfindungsgemäßen Verbindungen können entweder alleine oder in Formulierung als Arzneimittel zur Behandlung von Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, zur Unterstützung der narbenfreien Wundheilung, bei Altersflecken und bei Kontaktdermatitis zum Einsatz kommen.

Bei der Behandlung von Verletzungen des Nervengewebes kann mit den erfindungsgemäßen Verbindungen eine schnelle Narbenbildung an den Verletzungsstellen verhindert werden, d. h. es wird verhindert, daß die Narbenbildung eintritt, bevor die Axone wieder Verbindung miteinander aufnehmen. Damit würde eine Rekonstruktion der Nervenverbindungen erleichtert.

Ferner kann mit den erfindungsgemäßen Verbindungen die Ascites-Bildung bei Patienten unterdrückt werden. Ebenso lassen sich VEGF bedingte Ödeme unterdrücken.

Die Lymphangiogenese spielt eine wichtige Rolle bei der lymphogenen Metastasierung (Karpanen, T. et al., Cancere Res. 2001 Mar 1, 61 (5): 1786-90, Veikkola T. et al., EMBO J. 2001, Mar 15; 20 (6): 1223-31).

Die erfindungsgemäßen Verbindungen zeigen nun ebenfalls hervorragende Wirkung als VEGFR Kinase 3 - Inhibitoren und eignen sich daher auch als wirksame Inhibitoren der Lymphangiogenese.
Durch eine Behandlung mit den erfindungsgemäßen Verbindungen wird nicht nur eine Reduzierung der Größenentwicklung von Metastasen, sondern auch eine Verringerung der Anzahl der Metastasen erreicht.

Derartige Arzneimittel, deren Formulierungen und Verwendungen sind ebenfalls Gegenstand der vorliegenden Erfindung.

Die Erfindung betrifft somit ferner die Verwendung der Verbindungen der allgemeinen Formel I, zur Herstellung eines Arzneimittels zur Verwendung als, bzw. zur Behandlung von Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, als Immunsuppressiva, als Unterstützung bei der narbenfreien Wundheilung, bei Altersflecken und bei Kontaktdermatitis.

Ferner kann mit den erfindungsgemäßen Verbindungen die Ascites-Bildung bei Patienten unterdrückt werden. Ebenso lassen sich VEGF bedingte Ödeme unterdrücken.

Zur Verwendung der Verbindungen der Formel I als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff oder bei Bedarf ein oder mehrere Geschmacksstoffe beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.
Die oben beschrieben Formulierungen und Darreichungsformen sind ebenfalls Gegenstand der vorliegenden Erfindung.

Die Herstellung der erfindungsgemäßen Verbindungen erfolgt nach an sich bekannten Methoden. Beispielsweise gelangt man zu Verbindungen der Formel I dadurch, daß man
a) in einer Verbindung der allgemeinen Formel II 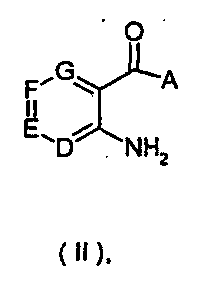 worin D bis G die in der allgemeinen Formel I angegebenen Bedeutungen haben und A für die Gruppe OR¹³ steht, wobei R¹³ für Wasserstoff, C₁-C₄-Alkyl oder C₁-C₆-Acyl steht, zuerst die Amin-Gruppe alkyliert und dann COA in ein Amid überführt; oder die NH₂-Gruppe in Halogen überführt, A in ein Amid überführt und Halogen in das entsprechende Amin überführt sowie gegebenenfalls eine Schutzgruppe abspaltet und ein Halogen in Cyanid überführt oder ein N-Oxid in ein Nitril überführt, oder
b) eine Verbindung der allgemeinen Formel III 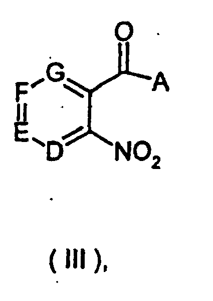 worin D bis G die in der allgemeinen Formel I angegebenen Bedeutungen haben und A für Halogen oder die Gruppe OR¹³ steht, wobei für R¹³ Wasserstoff, C₁-C₆-Alkyl oder C₁-C₆-Acyl steht, COA in ein Amid überführt, die Nitrogruppe zum Amin reduziert und dann alkyliert und gegebenenfalls einen Heterocyclus in ein N-Oxid überführt,
   oder
c) eine Verbindung der allgemeinen Formel IV 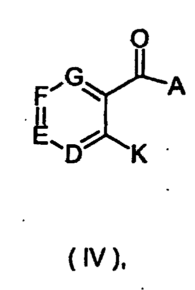 worin D bis G die in der allgemeinen Formel I angegebenen Bedeutungen haben, und K für Hydroxy oder Halogen steht, und A für Halogen oder für die Gruppe OR¹³ steht, wobei R¹³ für Wasserstoff, C₁-C₆-Alkyl oder C₁-C₆-Acyl stehen kann, K in ein Amin überführt, COA in ein Amid überführt oder, wenn K für Hydroxy steht, es in Halogen überführt und dann wie oben weiterverfährt.
   oder
d) eine Verbindung der allgemeinen Formel V zuerst alkyliert und dann das Anhydrid in das Amid überführt.

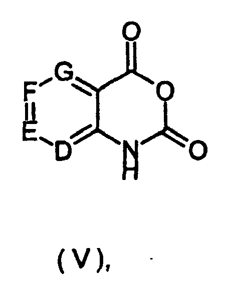

Die Reihenfolge der Verfahrensschritte kann in allen Fällen vertauscht werden.

Die Amidbildung erfolgt nach literaturbekannten Methoden.
Zur Amidbildung kann man von einem entsprechenden Ester ausgehen. Der. Ester wird nach J. Org. Chem. 1995, 8414 mit Aluminiumtrimethyl und dem entsprechenden Amin in Lösungsmitteln wie Toluol bei Temperaturen von 0°C bis zum Siedepunkt des Lösungsmittels umgesetzt. Enthält das Molekül zwei Estergruppen, werden beide in das gleiche Amid überführt.

Beim Einsatz von Nitrilen statt des Esters erhält man unter analogen Bedingungen Amidine.

Zur Amidbildung stehen aber auch alle aus der Peptidchemie bekannten Verfahren zur Verfügung. Beispielsweise kann die entsprechende Säure in aprotischen polaren Lösungsmitteln wie zum Beispiel Dimethylformamid über eine aktiviertes Säurederivat, zum Beispiel erhältlich mit Hydroxybenzotriazol und einem Carbodiimid wie zum Beispiel Diisopropylcarbodiimid oder auch mit vorgebildeten Reagenzien wie zum Beispiel HATU (Chem. Comm. 1994, 201) oder BTU, bei Temperaturen zwischen 0°C und dem Siedepunkt des Lösungsmittels mit dem Amin umgesetzt werden. Für die Amidbildung kann auch das Verfahren über das gemischte Säureanhydrid, das Säurechlorid, das Imidazolid oder das Azid eingesetzt werden. Bei Umsetzungen des Säurechlorids ist als Lösungsmittel Dimethylacetamid bei Temperaturen von Raumtemperatur bis zum Siedepunkt des Lösungsmittels vorzugsweise bei 80-100°C bevorzugt.

Sollen verschiedene Amidgruppen in das Molekül eingeführt werden, muss beispielsweise die zweite Estergruppe nach der Erzeugung der ersten Amidgruppe in das Molekül eingeführt und dann amidiert werden oder man hat ein Molekül in dem eine Gruppe als Ester, die andere als Säure vorliegt und amidiert die beiden Gruppen nacheinander nach verschiedenen Methoden.

Thioamide sind aus den Anthranilamiden durch Umsetzung mit Diphosphadithianen nach Bull Soc.Chim.Belg. 87,229,1978 oder durch Umsetzung mit Phosphorpentasulfid in Lösungsmitteln wie Pyridin oder auch ganz ohne Lösungsmittel bei Temperaturen von 0°C bis 200°C zu erhalten.

Die Reduktion der Nitrogruppe wird in polaren Lösungsmitteln bei Raumtemperatur oder erhöhter Temperatur durchgeführt. Als Katalysatoren für die Reduktion sind Metalle wie Raney-Nickel oder Edelmetallkatalysatoren wie Palladium oder Platin oder auch Palladiumhydroxid gegebenenfalls auf Trägem geeignet. Statt Wasserstoff können auch zum Beispiel Ammoniumformiat, Cyclohexen oder Hydrazin in bekannter Weise benutzt werden. Reduktionsmittel wie Zinn-II-chlorid oder Titan-(III)-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfalls unter Zusatz eines Lösungsmittels wie zum Beispiel Wasser, Methanol, Eisen/ Ammoniak etc. durchgeführt. Bei verlängerter Reaktionszeit kann bei dieser Variante eine Acylierung der Aminogruppe eintreten.

Wird eine Alkylierung einer Aminogruppe gewünscht, so kann man das Amin einer reduktiven Alkylierung mit Aldehyden oder Ketonen unterwerfen, wobei man in Gegenwart eines Reduktionsmittels wie beispielsweise Natriumcyanoborhydrid in einem geeigneten inerten Lösungsmittel wie zum Beispiel Ethanol bei Temperaturen von 0°C bis zum Siedepunkt des Lösungsmittels umsetzt. Wenn man von einer primären Aminogruppe ausgeht, so kann man gegebenenfalls nacheinander mit zwei verschiedenen Carbonylverbindungen umsetzen, wobei man gemischte Derivate erhält [Literatur z.B. Verardo et al. Synthesis (1993), 121; Synthesis (1991), 447; Kawaguchi, Synthesis (1985), 701; Micovic et al. Synthesis (1991), 1043]. Es kann vorteilhaft sein, zunächst die Schiffsche Base durch Umsetzung des Aldehyds mit dem Amin in Lösungsmitteln wie Ethanol oder Methanol, gegebenenfalls unter Zugabe von Hilfsstoffen wie Eisessig zu bilden und dann erst Reduktionsmittel wie z. B. Natriumcyanoborhydrid zuzusetzen. Ein N-Oxid übersteht diese Reaktionsbedingungen.

Eine Alkylierung kann man auch dadurch erreichen, daß man nach der Mitsonubo Variante mit einem Alkohol in Gegenwart von beispielsweise Triphenylphosphin und Azodicarbonsäureester umsetzt. Eine Alkylierung der Aminogruppe, kann aber auch durch Alkylierungsmittel wie Halogenide, Tosylate, Mesylate oder Triflate erfolgen. Als Lösungsmittel eignen sich beispielsweise polare Lösungsmittel wie Ethanol, Tetrahydrofuran, Acetonitril oder Dimethylformamid. Der Zusatz einer Hilfsbase wie Triethylamin, DABCO Pyridin oder Kaliumcarbonat kann vorteilhaft sein.
Da bei einer freien Aminogruppe die Gefahr einer Doppelalkylierung besteht, kann vorteilhaft Isatosäureanhydrid eingesetzt werden. Mit Basen wie Natriumhydrid aber auch Cäsiumkarbonat in Lösungsmitteln wie Tetrahydrofuran oder Dimethylformamid bei Temperaturen zwischen Raumtemperatur und dem Siedepunkt des Lösungsmittels, vorzugsweise bei 60°C, lässt es sich in das Anion überführen, das dann mit dem Alkylierungsmittel weiter umgesetzt wird.

Etherspaltungen werden nach literaturüblichen Verfahren durchgeführt. Dabei kann auch bei mehreren im Molekül vorhandenen Gruppen eine selektive Spaltung erreicht werden. Dabei wird der Ether beispielsweise mit Bortribromid in Lösungsmitteln wie Dichlormethan bei Temperaturen zwischen -100 °C bis zum Siedepunkt des Lösungsmittels vorzugsweise bei -78 °C behandelt. Es ist aber auch möglich, den Ether durch Natriumthiomethylat in Lösungsmitteln wie Dimethylformamid zu spalten. Die Temperatur kann zwischen Raumtemperatur und dem Siedepunkt des Lösungsmittels vorzugsweise bei 150°C liegen. Bei Benzyläthem gelingt die Spaltung auch mit starken Säuren wie zum Beispiel Trifluoressigsäure bei Temperaturen von Raumtemperatur bis zum Siedepunkt.

Die Umwandlung einer Hydroxygruppe, die ortho- oder paraständig zu einem Stickstoff eines 6-Ringhetaryls steht, in Halogen, kann beispielsweise durch Umsetzen mit anorganischen Säurehalogeniden wie zum Beispiel Phosphoroxychlorid, gegebnenfalls in einem inerten Lösungsmittel, bei Temperaturen bis zum Siedepunkt des Lösungsmittels oder des Säurehalogenids durchgeführt werden.

Die Substitution eines Halogens, Tosylates, Triflates oder Nonaflates , die ortho oder para zu einem Stickstoff in einem 6-gliedrigen Heteroaromaten stehen, gelingt durch Umsetzung mit einem entsprechenden Amin in inerten Lösungsmitteln wie beispielsweise Xylol oder in polaren Lösungsmitteln wie N-Methylpyrrolidon oder Dimethylacetamid bei Temperaturen von 60-170°C. Es ist aber auch Erhitzen ohne Lösungsmittel möglich. Der Zusatz einer Hilfsbase wie Kaliumcarbonat oder Cäsiumcarbonat oder der Zusatz von Kupfer und/oder Kupferoxid kann vorteilhaft sein. Bei nicht aktivierten Halogenen oder Triflaten ist nach J.org.Chem. 2000,1158 eine Palladium-katalysierte Einführung des Aminteils möglich. Als Base dient vorzugsweise Natrium-t-butylat, als Hilfsligang ein Biphenylphosphin.

Die Einführung der Halogene Chlor, Brom oder Jod über eine Aminogruppe kann beispielsweise auch nach Sandmeyer erfolgen, indem man die mit Nitriten intermediär gebildeten Diazoniumsalze mit Kupfer(I)chlorid oder Kupfer(I)bromid in Gegenwart der entsprechenden Säure wie Salzsäure oder Bromwasserstoffsäure oder mit Kaliumjodid umsetzt.

Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid. Die Entfernung der Aminogruppe kann entweder durch Umsetzung mit einem organischen Salpetrigsäureester in Tetrahydrofuran oder durch Diazotierung und reduktive Verkochung des Diazoniumsalzes beispielsweise mit phosphoriger Säure gegebenenfalls unter Zugabe von Kupfer (I) oxid bewerkstelligt werden.

Die Einführung von Fluor gelingt beispielsweise durch Balz-Schiemann-Reaktion des Diazoniumtetrafluorborates oder nach J. Fluor. Chem. 76, 1996, 59-62 durch Diazotierung i.G. von HFxPyridin und anschliessende Verkochung gegebenenfalls i.G. einer Fluoridionenquelle wie z.B. Tetrabutylammoniumfluorid.

Die Abspaltung der t-Butoxycarbonylgruppe erfolgt bekanntermassen dadurch, daß man in einem Lösungsmittel wie Tetrahydrofuran, Dioxan oder Ethanol mit einer Säure wie zB 1N-Salzsäure bei Temperaturen zwischen Raumtemperatur und dem Siedepunkt des Lösungsmittels umsetzt. Es ist auch möglich, die t-BOC-Gruppe mit starkan Säuren wie Trifluoressigsäure bei Temperaturen zwischen -20°C bis zum Siedepunkt, vorzugswesie bei Raumtemperatur abzuspalten. Ein Lösungsmittel wie Methylenchlorid ist nicht unbedingt erforderölic, kann aber vorteilhaft sein.

Die Acylierung eines Amins erfolgt in bekannter Weise entweder nach den unter Amidbildung beschriebenen Verfahren oder durch Umsetzung mit aktivierten Säurederivaten wie beispielsweise Säurechlorid oder Säureanhydrid in Lösungsmitein wie Methylenchlorid, Actonitril oder Tetrahydrofuran gegebenenfalls in Gegenwart von Basen wie Triethylamin. Ein Zusaz von katalytischen Mengen Dimethylaminopyridin kann vorteilhaft sein.

Die Einführung einer Nitrilgruppe erfolgt nach literaturbekannten Methoden. So kann durch schwermetall katalysierte Reaktion Fluchtgruppen wie Halogene, Tosylate, Mesylate oder Triflate gegen Nitril ersetzt werden. Als Katalysator eignen sich Palladium (0) oder Palladium(II) Katalysatoren. Als Cyanidquelle dient beispielsweise Zink(II)cyanid. Als Lösungsmittel dienen beispielsweise Dimethylacetamid oder Dimethylformamid. Ein Zusatz von Zinkpulver und Bis (diphenylphosphino)ferrocen sind für die Reaktion vorteilhaft. Auch Kupfer(I)-cyanid kann eingesetzt werden. Als Lösungsmittel eignen sich beispielsweise dipolare aprotische Lösungsmittel wie Dimethylformamid.

Die lsomerengemische können nach üblichen Methoden wie beispielsweise Kristallisation, jede Form von Chromatographie oder Salzbildung in die Enantiomeren bzw. E/Z-tsomeren aufgetrennt werden.

Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

Die nachfolgenden Beispiele erläutern die Herstellung der erfindungsgemäßen Verbindungen, ohne den Umfang der beanspruchten Verbindungen auf diese Beispiele zu beschränken.

### Beispiel 1

### Herstellung von N-(Isochinolin-3-yl)-2-(4-(2-cyanopyridylmethyl)aminobenzoesäureamid

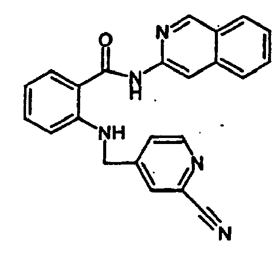

920 mg (2,5 mMol) N-(Isochinolin-3-yl)-2-(4-pyridytmethyl)aminobenzoesäureamid-N-oxid werden in einem Glasdruckgefäss nacheinander mit 20 ml Dimethylformamid nacheinander mit 760 mg (7,5 mMol) Triethylamin und 1,24 g (12,5 mMol) Trimethylsilylcyanid versetzt und dann für 10 Stunden auf 110 °C Badtemperatur erwärmt. Es wird dann mit Wasser auf ca 200 ml verdünnt und dreimal mit je 50 ml Essigester ausgeschüttelt. Die gesammelte organische Phase wird mit 50 ml Wasser gewaschen, getrocknet, filtriert und eingeengt. Der Rückstand wird zunächst über Kieselgel mit Essigester:Hexan=1:1 und anschliessend nochmals über Kieselgel mit Dichlormethan:Ethanol=100:2 als Elutionsmittel chromatographiert. Man erhält 132 mg (14% der Theorie) an N-(Isochinolin-3-yl)-2-(4-2-cyanopyridylmethyl)aminobenzoesäureamid als Harz

In analoger Verfahrensweise werden auch folgende Verbindungen hergestellt: 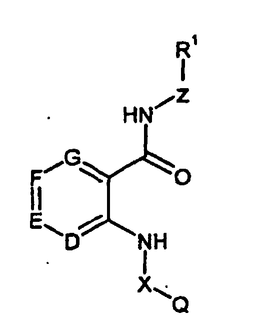
D steht für die Gruppe C-R³
E steht für die Gruppe C-R⁴
F steht für die Gruppe C-R⁵
G steht für die Gruppe C-R⁶
und R³, R⁴, R⁵, R⁶ jeweils für Wasserstoff stehen

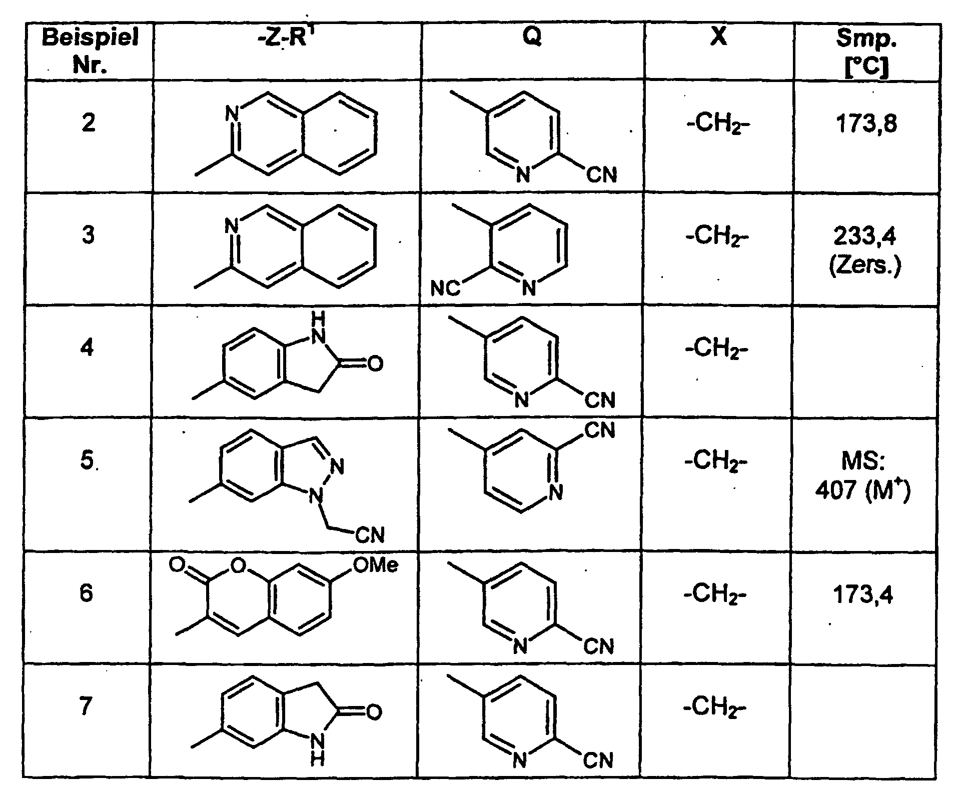

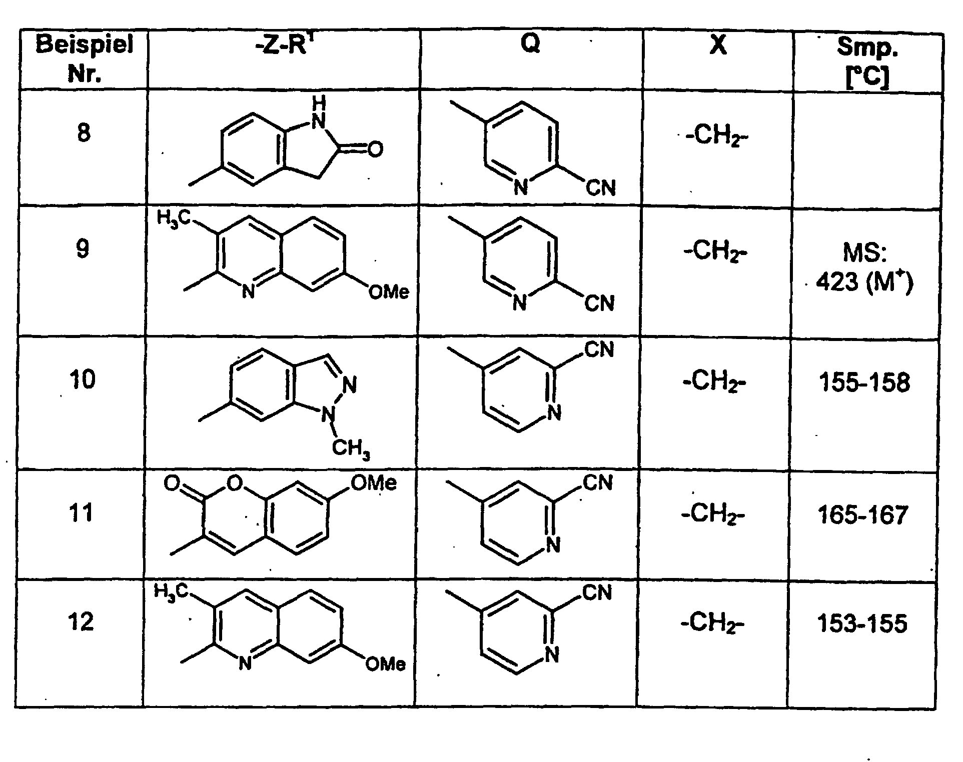

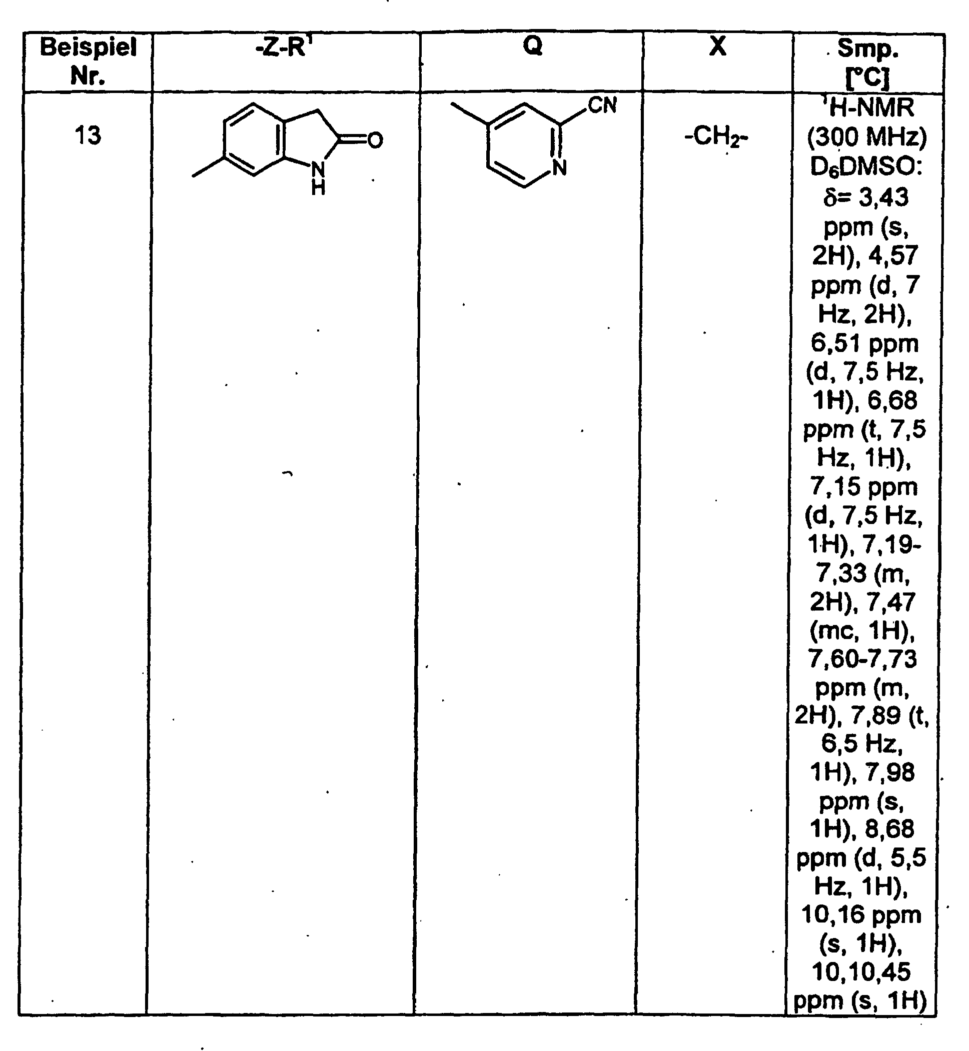

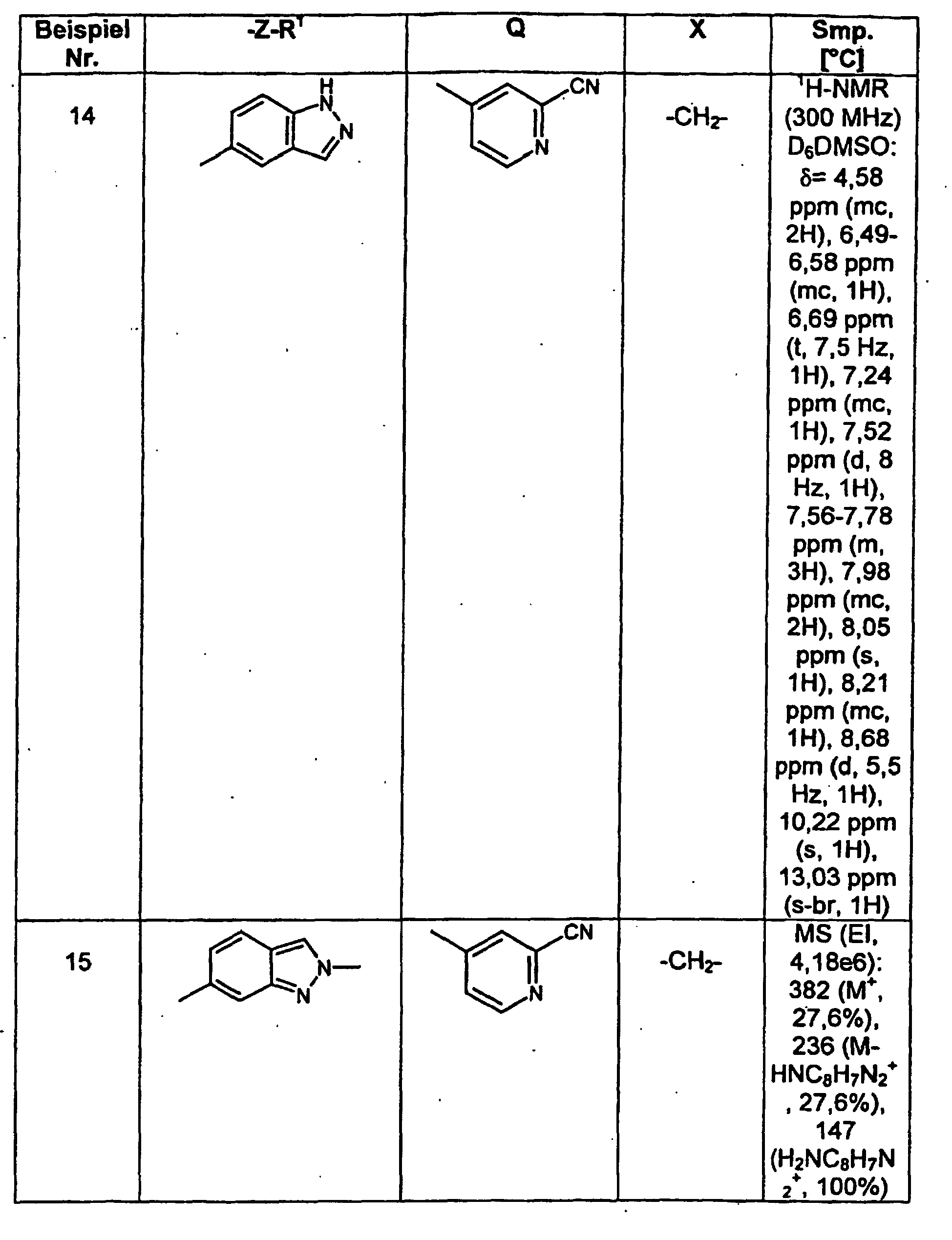

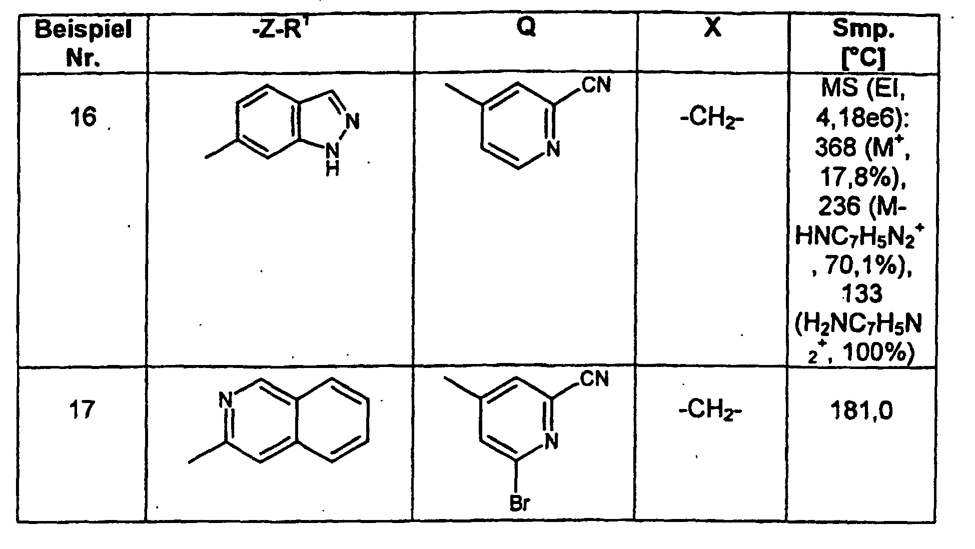

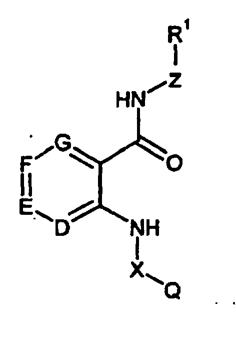

D steht für ein Stickstoffatom
E steht für die Gruppe C-R⁴
F steht für die Gruppe C-R⁵
G steht für die Gruppe C-R⁶
und R⁴, R⁵, R⁶ jeweils für Wasserstoff stehen

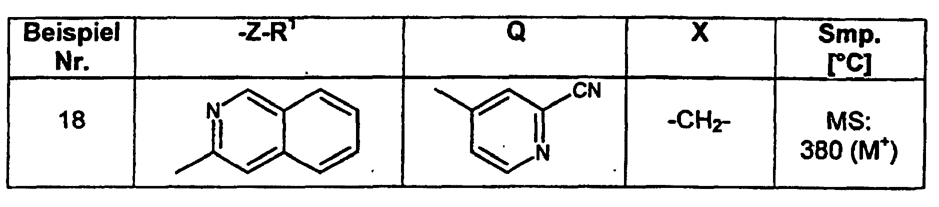

Soweit die Herstellung der Zwischenverbindungen nicht beschrieben wird, sind diese bekannt oder analog zu bekannten Verbindungen oder hier beschriebenen Verfahren herstellbar.

Die nachfolgenden Anwendungsbeispiele erläutern die biologische Wirkung und Verwendung der erfindungsgemäßen Verbindungen ohne diese auf die Beispiele zu beschränken.

**Für die Versuche benötigte Lösungen**
Stammlösungen
Stammlösung A: 3mM ATP in Wasser pH 7,0 (-70°C)
Stammlösung B: g-33P-ATP 1mCi/ 100µl
Stammlösung C: poly-(Glu4Tyr) 10mg/ ml in Wasser

Lösung für Verdünnungen
Substratlösemittel: 10mM DTT, 10 mM Manganchlorid, 100 mM Magnesiumchlorid
Enzymlösung: 120 mM Tris/ HCl, pH 7,5, 10 µM Natriumvanadiumoxid

### Anwendungsbeispiel 1

### Hemmung der KDR- und FLT-1 Kinaseaktivität in Gegenwart der erfindungsgemäßen Verbindungen

In einer spitz zulaufenden Mikrotiterplatte (ohne Proteinbindung) werden 10 µl Substratmix (10µl Vol ATP Stammlösung A + 25µCi g-33P-ATP (ca. 2,5 µl der Stammlösung B) + 30µl poly-(Glu4Tyr) Stammlösung C + 1,21ml Substratlösemittel), 10 µl Hemmstofflösung (Substanzen entsprechend den Verdünnungen, als Kontrolle 3% DMSO in Substratlösemittel) und 10 µl Enzymlösung (11,25µg Enzymstammlösung (KDR oder FLT-1 Kinase) werden bei 4°C in 1,25ml Enzymlösung verdünnt) gegeben. Es wird gründlich durchgemischt und bei 10 Minuten Raumtemperatur inkubiert. Anschließend gibt man 10µl Stop-Lösung (250mM EDTA, pH 7,0) zu, mischt und überträgt 10 µl der Lösung auf einen P 81 Phosphozellulosefilter. Anschließend wird mehrfach in 0,1 M Phosphorsäure gewaschen. Das Filterpapier wird getrocknet, mit Meltilex beschichtet und im Microbetazähler gemessen.

Die IC50-Werte bestimmen sich aus der Inhibitorkonzentration, die notwendig ist, um den Phosphateinbau auf 50% des ungehemmten Einbaus nach Abzug des Leerwertes (EDTA gestoppte Reaktion) zu hemmen.

Die Ergebnisse der Kinase-Inhibition IC50 in µM sind in der nachfolgenden Tabelle dargestellt:

| **Beispiel Nr.** | **VEGFR II (KDR) [µM]** |
|---|---|
| 1 | 0,01 |
| 2 | 0,05 |
| 3 | 0,05 |
| 4 | 0,3 |
| 5 | 0,3 |
| 6 | 0,3 |
| 10 | 0,2 |
| 11 | 0,2 |
| 12 | 0,7 |
| 13 | 0,2 |
| 14 | 0,2 |
| 15 | 0,2 |
| 16 | 0,1 |
| 18 | 0,02 |

### Anwendungsbeispiel 2

### Cytochrom P450 - Inhibition

Die Cytochrom P450 - Inhibition wurde entsprechend der Veröffentlichung von Crespi et al. (Anal. Biochem., 248, 188-190 (1997)) unter Verwendung von Baculovirus/ Insektenzellen-exprimierten, humanen Cytochrom P 450 Isoenzymen ( 1A2, 2C9, 2C19, 2D6, 3A4) durchgeführt.

Die Ergebnisse sind in der folgenden Tabelle dargestellt.

### Hemmung der Cytochrom P450 Isoenzyme (IC50, µM)

| Cytochrom P450 Isoenzym | 1A2 | 2C9 | 2C19 | 2D6 | 3A4 |
|---|---|---|---|---|---|
| Beispiel 2.54 der WO 00/27819 | 5,2 | 0,2 | 0,05 | > 30 | 3,6 |
| Beispiel 1 | > 30 | 1,2 | 1,8 | > 30 | > 30 |

Aus dem Ergebnis ist deutlich die überlegene Wirkung der erfindungsgemäßen Verbindungen gegenüber den bekannten Verbindungen zu erkennen.

## Patentansprüche

1. Verbindungen der allgemeinen Formel I 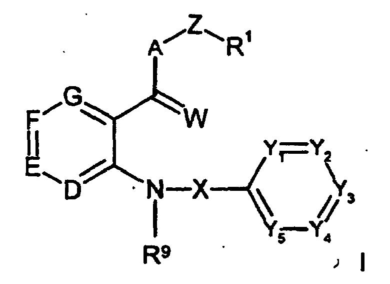 in der
A für die Gruppe -N(R⁷)- steht,
W für Sauerstoff, Schwefel, zwei Wasserstoffatome oder die Gruppe -N(R⁸)- steht,
Z für eine Bindung, die Gruppe -N(R¹⁰)- oder =N-, für verzweigtes oder unverzweigtes C₁-C₁₂₋Alkyl oder für die Gruppe 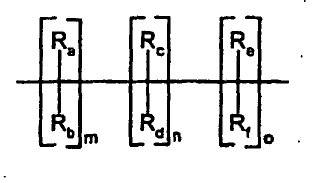 steht,
m, n und o für 0 - 3 stehen,
R_{a,} R_{b}, R_{c}, R_{d}, Rₑ, R_{f} unabhängig voneinander für Wasserstoff, Fluor, C₁-C₄ Alkyl oder die Gruppe -N(R¹¹)- stehen und/ oder Rₐ und/ oder R_{b} mit R_{c} und/ oder R_{d} oder R_{c} mit Rₑ und/ oder R_{f} eine Bindung bilden können, oder bis zu zwei der Reste Rₐ-R_{f} eine Brücke mit je bis zu 3 C-Atomen zu R¹ oder zu R⁷ schließen können,
X für C₁-C₆-Alkyl steht,
R¹ für gegebenenfalls ein- oder mehrfach, gleich oder verschieden mit Halogen, Hydroxy, C₁-C₆-Alkyloxy, Aralkyloxy, C₁-C₆-Alkyl und/ oder mit der Gruppe-NR¹²R¹³ substituiertes verzweigtes oder unverzweigtes C₁-C₁₂-Alkyl oder C₂-C₁₂-Alkenyl steht; oder für gegebenenfalls ein- oder mehrfach, gleich oder verschieden mit Halogen, Hydroxy, C₁₋C₆-Alkyloxy, C₁-C₆-Alkyl und/ oder mit der Gruppe-NR¹²R¹³ substituiertes C₃-C₁₀-Cycloalkyl oder C₃₋C₁₀-Cycloalkenyl steht; oder für gegebenenfalls ein- oder mehrfach, gleich oder verschieden mit Halogen, Hydroxy, C₁-C₆-Alkyloxy, Aralkyloxy, C₁₋C₆-Alkyl, Halo-C₁-C₆-alkyl, Cyano-C₁-C₆-alkyl oder mit der Gruppe =O, OR¹⁴ oder R¹⁴ substituiertes Aryl oder Hetaryl steht,
Y₁ bis Y₅ jeweils für ein Stickstoffatom oder für die Gruppe -CY⁶ stehen,
Y⁶ für Cyano, Halogen, C₁-C₆-Alkyl, C₁-C₆-Alkoxy, Amino oder Hydroxy steht, wobei im Ring mindestens ein Stickstoff-Atom enthalten ist und am Ring mindestens ein Cyano-Rest enthalten ist,
D für ein Stickstoffatom oder für die Gruppe C-R³ steht,
E für ein Stickstoffatom oder für die Gruppe C-R⁴ steht,
F für ein Stickstoffatom oder für die Gruppe C-R⁵ steht,
G für ein Stickstoffatom oder für die Gruppe C-R⁶ steht, wobei
R³,R⁴,R⁵ und R⁶ für Wasserstoff, Halogen oder unsubstituiertes oder gegebenenfalls ein- oder mehrfach mit Halogen substituiertes C₁-C₆-Alkoxy, C₁-C₆-Alkyl oder C₁-C₆₋Carboxyalkyl stehen,
R⁷ für Wasserstoff oder C₁-C₆-Alkyl steht oder mit Rₐ₋R_{f} von Z oder zu R¹ eine Brücke mit bis zu 3 Ringgliedem bildet,
R⁸, R⁹ R¹⁰ und R¹¹ für Wasserstoff oder C₁-C₆-Alkyl stehen,
R¹² und R¹³ für Wasserstoff, C₁-C₆-Alkyl stehen oder einen Ring bilden,der ein weiteres Heteroatom enthalten kann,
R¹⁴ für die Gruppe C₁-C₁₅-Alkyl-R¹⁵, in der der Alkylrest ein- oder mehrfach durch Sauerstoff unterbrochen sein kann, oder für die Gruppe (CH₂-CH₂-O)ᵤ(CH₂)ᵥ₋R¹⁵ steht,
R¹⁵ für Aryl, Hetaryl, C₁-C₆-Alkyl, Aralkyl, -CH₂CN oder für die Gruppe NR¹⁶R¹⁷ steht,
R¹⁶ und R¹⁷ für Wasserstoff, C₁-C₆-Alkyl, C₁-C₆-Acyl stehen oder einen Ring bilden,der ein weiteres Heteroatom enthalten kann, und
u und v für 0-5 stehen, bedeuten, sowie deren Isomeren,
Enantiomeren und Salze.

2. Verbindungen der allgemeinen Formel I, gemäß Anspruch 1, in der
A für die Gruppe -N(R⁷)- steht,
W für Sauerstoff steht,
Z für eine Bindung oder für verzweigtes oder unverzweigtes C₁-C₁₂-Alkyl steht,
X für C₁-C₆-Alkyl steht,
R¹ für gegebenenfalls ein- oder mehrfach, gleich oder verschieden mit Halogen, Hydroxy, C₁-C₆-Alkyloxy, Aralkyloxy, C₁-C₆-Alkyl und/ oder mit der Gruppe-NR¹²R¹³ substituiertes verzweigtes oder unverzweigtes C₁-C₁₂-Alkyl oder C₂-C₁₂-Alkenyl steht; oder für gegebenenfalls ein- oder mehrfach, gleich oder verschieden mit Halogen, Hydroxy, C₁₋C₆-Alkyloxy, C₁-C₆-Alkyl und/ oder mit der Gruppe -NR¹²R¹³ substituiertes C₃-C₁₀-Cycloalkyl oder C₃₋C₁₀-Cycloalkenyl steht; oder für gegebenenfalls ein- oder mehrfach, gleich oder verschieden mit Halogen, Hydroxy, C₁-C₆-Alkyloxy, Aralkyloxy, C₁₋C₆-Alkyl, Halo-C₁-C₆-alkyl, Cyano-C₁-C₆-alkyl oder mit der Gruppe =O, OR¹⁴ oder R¹⁴ substituiertes Aryl oder Hetaryl steht,
Y₁ bis Y₅ jeweils für ein Stickstoffatom oder für die Gruppe -CY⁶ stehen,
Y⁶ für Cyano, Halogen, C₁-C₆-Alkyl, C₁-C₆-Alkoxy, Amino oder Hydroxy steht, wobei im Ring mindestens ein Stickstoff-Atom enthalten ist und am Ring mindestens ein Cyano-Rest enthalten ist,
D für ein Stickstoffatom oder für die Gruppe C-R³ steht,
E für ein Stickstoffatom oder für die Gruppe C-R⁴ steht,
F für ein Stickstoffatom oder für die Gruppe C-R⁵ steht,
G für ein Stickstoffatom oder für die Gruppe C-R⁶ steht, wobei
R³,R⁴ ,R⁵ und R⁶ für Wasserstoff, Halogen oder unsubstituiertes oder gegebenenfalls ein- oder mehrfach mit Halogen substituiertes C₁-C₆-Alkoxy, C₁-C₆-Alkyl oder C₁-C₆₋Carboxyal-kyl stehen,
R⁷ für Wasserstoff oder C₁-C₆-Alkyl steht oder mit Rₐ₋R_{f} von Z oder zu R¹ eine Brücke mit bis zu 3 Ringgliedem bildet,
R⁹ für Wasserstoff oder C₁-C₆-Alkyl steht,
R¹² und R¹³ für Wasserstoff, C₁-C₆-Alkyl stehen oder einen Ring bilden,der ein weiteres Heteroatom enthalten kann,
R¹⁴ für die Gruppe (CH₂-CH₂-O)ᵤ(CH₂)ᵥ-R¹⁵ steht,
R¹⁵ für Aryl, Hetaryl, C₁₋₆-Alkyl, Aralkyl, -CH₂CN oder für die Gruppe NR¹⁶R¹⁷ steht,
R¹⁶ und R¹⁷ für Wasserstoff, C₁-C₆-Alkyl, C₁-C₆-Acyl stehen oder einen Ring bilden,der ein weiteres Heteroatom enthalten kann, und
u und v für 0-5 stehen, bedeuten, sowie deren Isomeren, Enantiomeren und Salze.

3. Verbindungen der allgemeinen Formel I, gemäß den Ansprüchen 1 und 2, in der
A für die Gruppe -N(R⁷)- steht,
W für Sauerstoff steht,
Z für eine Bindung oder für verzweigtes oder unverzweigtes C₁-C₁₂-Alkyl steht,
X für C₁-C₆-Alkyl steht,
R¹ für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl, Cyano-C₁-C₆-alkyl, oder mit der Gruppe =O, OR¹⁴ oder R¹⁴ substituiertes Aryl oder Hetaryl steht,
Y₁ bis Y₅ gemeinsam für einen Pyridyl-Ring stehen, der mit einer Cyanogruppe substituiert ist und der zusätzlich mit Halogen, C₁-C₆-Alkyl, C₁-C₆-Alkoxy, Amino, C₁-C₆-Carboxyalkylamino oder Hydroxy substituiert sein kann,
D für ein Stickstoffatom oder für die Gruppe C-R³ steht,
E für ein Stickstoffatom oder für die Gruppe C-R⁴ steht,
F für ein Stickstoffatom oder für die Gruppe C-R⁵ steht,
G für ein Stickstoffatom oder für die Gruppe C-R⁶ steht, wobei
R³,R⁴ ,R⁵ und R⁶ für Wasserstoff, Halogen oder unsubstituiertes oder gegebenenfalls ein- oder mehrfach mit Halogen substituiertes C₁-C₆-Alkoxy, C₁-C₆-Alkyl oder C₁-C₆₋Carboxyalkyl stehen,
R⁷ für Wasserstoff oder C₁-C₆-Alkyl steht oder mit Rₐ₋R_{f} von Z oder zu R¹ eine Brücke mit bis zu 3 Ringgliedem bildet,
R⁹ für Wasserstoff oder C₁-C₆-Alkyl steht,
R¹⁴ für die Gruppe (CH₂-CH₂-O)ᵤ(CH₂)ᵥ-R¹⁵ steht,
R¹⁵ für Aryl, Hetaryl, C₁₋₆-Alkyl, Aralkyl, -CH₂CN oder für die Gruppe NR¹⁶R¹⁷ steht,
R¹⁶ und R¹⁷ für Wasserstoff, C₁-C₆-Alkyl, C₁-C₆-Acyl stehen oder einen Ring bilden,der ein weiteres Heteroatom enthalten kann, und
u und v für 0-5 stehen, bedeuten, sowie deren Isomeren,
Enatiomeren und Salze.

4. Verbindungen der allgemeinen Formel I, gemäß den Ansprüchen 1 bis 3, in der
A für die Gruppe -N(R⁷)-steht,
W für Sauerstoff steht,
Z für eine Bindung oder C₁-C₆-Alkyl steht,
X für C₁-C₆-Alkyl steht,
R¹ für gegebenenfalls ein- oder mehrfach, gleich oder verschieden mit Halogen, Hydroxy, C₁-C₆-Alkyloxy, Aralkyloxy, C₁-C₆-Alkyl, Halo-C₁-C₆-alkyl, Cyano-C₁-C₆-alkyl oder mit der Gruppe =O substituiertes Thiophen, Furan, Oxazol, Thiazol, Indazolyl, Imidazol, Pyrazol, Pyridin, Pyrimidin, Triazin, Chinolin, Isochinolin oder 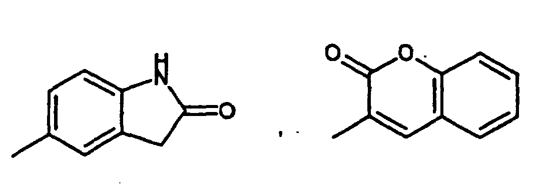 oder 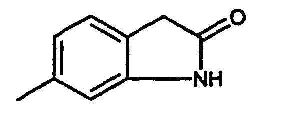 steht,
Y₁ bis Y₅ gemeinsam für einen Pyridyl-Ring stehen, der mit einer Cyanogruppe substituiert ist und der zusätzlich mit Halogen substituiert sein kann,
D für ein Stickstoffatom oder für die Gruppe C-R³ steht,
E für die Gruppe C-R⁴ steht,
F für die Gruppe C-R⁵ steht,
G für die Gruppe C-R⁶ steht, wobei
R³, R⁴, R⁵ und R⁶ für Wasserstoff stehen,
R⁷ für Wasserstoff steht und
R⁹ für Wasserstoff steht, bedeuten, sowie deren
Isomeren, Enantiomeren und Salze.

5. Verbindungen der allgemeinen Formel 1, gemäß den Ansprüchen 1 bis 4, in der
A für die Gruppe -N(R⁷)- steht,
W für Sauerstoff steht,
Z für eine Bindung steht,
X für C₁-C₆-Alkyl steht,
R¹ für gegebenenfalls ein- oder mehrfach, gleich oder verschieden mit C₁-C₆-Alkyloxy, C₁-C₆-Alkyl, Cyano-C₁-C₆-alkyl oder mit der Gruppe =O substituiertes Indazolyl, Chinolin, Isochinolin oder 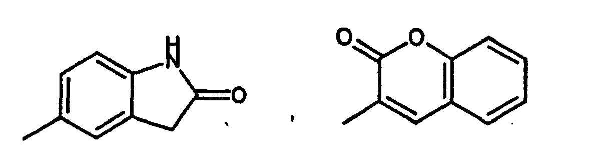 oder 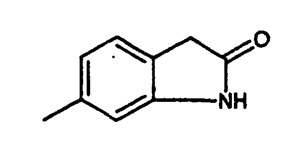 steht,
Y₁ bis Y₅ gemeinsam für einen Pyridyl-Ring stehen, der mit einer Cyanogruppe substituiert ist und der zusätzlich mit Halogen substituiert sein kann,
D für ein Stickstoffatom oder für die Gruppe C-R³ steht,
E für die Gruppe C-R⁴ steht,
F für die Gruppe C-R⁵ steht,
G für die Gruppe C-R⁶ steht, wobei
R³, R⁴, R⁵ und R⁶ für Wasserstoff stehen,
R⁷ für Wasserstoff steht und
R⁹ für Wasserstoff steht, bedeuten, sowie deren
Isomeren, Enantiomeren und Salze.

6. Arzneimittel, enthaltend mindestens eine Verbindung gemäß den Ansprüchen 1 bis 5.

7. Arzneimittel gemäß Anspruch 6, zur Verwendung bei Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotischen Erkrankungen, wie Leberzirrhose, mesangialzellproliferative Erkrankungen, Artheriosklerose, Verletzungen des Nervengewebes, Hemmung der Reocclusion von Gefäßen nach Ballonkatheterbehandlung, Gefäßprothetik oder Einsetzen von mechanischen Vorrichtungen zum Offenhalten von Gefäßen, wie z. B. Stents, und als Immunsuppressiva, und zur Unterstützung der narbenfreien Wundheilung, und bei Altersflecken und bei Kontaktdermatitis.

8. Verbindungen gemäß den Ansprüchen 1 bis 5 und Arzneimittel, gemäß den Ansprüchen 6 und 7, mit geeigneten Formulierungs und Trägerstoffen.

9. Verbindungen der Formel I, gemäß den Ansprüchen 1 bis 5, zur Verwendung als Inhibitoren der Tyrosinkinase KDR und FLT.

10. Verbindungen der allgemeinen Formel I, gemäß den Ansprüchen 1 bis 5, zur Verwendung in Form eines pharmazeutischen Präparats für die enteral, parenterale und orale Applikation.

11. Verbindungen gemäß den Ansprüchen 1 bis 5, zur Verwendung bei Psoriasis, Kaposis Sarkom, Restenose, wie z. B. Stent-induzierte Restenose, Endometriose, Crohns disease, Hodgkins disease, Leukämie, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose, Verletzungen des Nervengewebes und zur Hemmung der Reocclusion von Gefäßen nach Ballonkatheterbehandlung, bei der Gefäßprothetik oder nach dem Einsetzen von mechanischen Vorrichtungen zum Offenhalten von Gefäßen, wie z. B. Stents, und als Immunsuppressiva, undzur Unterstützung der narbenfreien Wundheüung, und bei Altersflecken und bei Kontaktdermatitis.

12. Verbindungen der allgemeinen Formel I, gemäß den Ansprüchen 1 bis 5, zur Verwendung als VEGFR Kinase 3 - Inhibitor der Lymphangiogenese.

## Claims

1. Compounds of general formula I 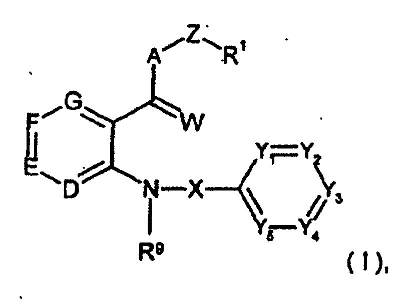 in which
A stands for the group -N(R⁷)-,
W stands for oxygen, sulphur, two hydrogen atoms or the group -N(R⁸)-,
Z stands for a bond, the group -N(R¹⁰)- or =N-, for branched or unbranched C₁-C₁₂-alkyl or for the group 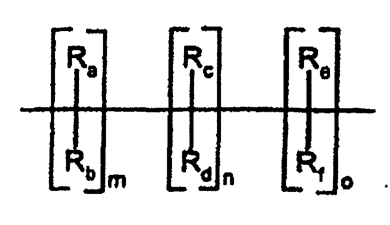 m, n and o stand for 0-3,
Rₐ, R_{b}, R_{c}, R_{d}, Rₑ, R_{f}, independently of one another, stand for hydrogen, fluorine, C₁-C₄-alkyl or the group -N(R¹¹)-, and/or Rₐ and/or R_{b} can form a bond with R_{c} and/or R_{d} or R_{c} can form a bond with Rₑ and/or R_{f}, or up to two of radicals Rₐ-R_{f} can close a bridge with up to 3 C atoms each to form R¹ or to form R⁷,
X stands for C₁-C₆-alkyl,
R¹ stands for branched or unbranched C₁-C₁₂-alkyl or C₂-C₁₂-alkenyl that is optionally substituted in one or more places in the same way or differently with halogen, hydroxy, C₁-C₆-alkyloxy, aralkyloxy, C₁-C₆-alkyl and/or with the group -NR¹²R¹³; or for C₃-C₁₀-cycloalkyl or C₃-C₁₀-cycloalkenyl that is optionally substituted in one or more places in the same way or differently with halogen, hydroxy, C₁-C₆-alkyloxy, C₁-C₆-alkyl and/or with the group -NR¹²R¹³; or for aryl or hetaryl that is optionally substituted in one or more places in the same way or differently with halogen, hydroxy, C₁-C₆₋alkyloxy, aralkyloxy, C₁-C₆-alkyl, halo-C₁-C₆-alkyl, cyano-C₁-C₆-alkyl or with the group =O OR¹⁴, or R¹⁴,
Y₁ to Y₅ in each case stand for a nitrogen atom or for the group -CY⁶,
Y⁶ stands for cyano, halogen, C₁-C₆-alkyl, C₁-C₆₋alkoxy, amino or hydroxy, wherein at least one nitrogen atom is contained in the ring and at least one cyano radical is contained on the ring,
D stands for a nitrogen atom or for the group C-R³,
E stands for a nitrogen atom or for the group C-R⁴,
F stands for a nitrogen atom or for the group C-R⁵,
G stands for a nitrogen atom or for the group C-R⁶, wherein
R³, R⁴, R⁵ and R⁶ stand for hydrogen, halogen, or C₁-C₆-alkoxy, C₁-C₆-alkyl or C₁-C₆-carboxyalkyl that is unsubstituted or that is optionally substituted in one or more places with halogen,
R⁷ stands for hydrogen or C₁-C₆-alkyl or forms a bridge with up to 3 ring members with Rₐ-R_{f} from Z or to form R¹,
R⁸, R⁹, R¹⁰ and R¹¹ stand for hydrogen or C₁-C₆₋alkyl,
R¹² and R¹³ stand for hydrogen, C₁-C₆-alkyl or form a ring that can contain another heteroatom,
R¹⁴ stands for the group C₁-C₁₅-alkyl-R¹⁵, in which the alkyl radical can be interrupted in one or more places by oxygen or for the group (CH₂-CH₂-O)ᵤ(CH₂)ᵥ-R¹⁵,
R¹⁵ stands for aryl, hetaryl, C₁-C₆-alkyl, aralkyl, -CH₂CN or for the group NR¹⁶R¹⁷,
R¹⁶ and R¹⁷ stand for hydrogen, C₁-C₆-alkyl, C₁-C₆₋acyl or form a ring that can contain another heteroatom, and
u and v stand for 0-5, as well as isomers, enantiomers and salts thereof.

2. Compounds of general formula I, according to Claim 1, in which
A stands for the group -N(R⁷)-,
W stands for oxygen,
Z stands for a bond or for branched or unbranched C₁-C₁₂-alkyl,
X stands for C₁-C₆-alkyl,
R¹ stands for branched or unbranched C₁-C₁₂-alkyl or C₂-C₁₂-alkenyl that is optionally substituted in one or more places in the same way or differently with halogen, hydroxy, C₁-C₆-alkyloxy, aralkyloxy, C₁-C₆-alkyl and/or with the group -NR¹²R¹³; or for C₃-C₁₀-cycloalkyl or C₃-C₁₀-cycloalkenyl that is optionally substituted in one or more places in the same way or differently with halogen, hydroxy, C₁-C₆-alkyloxy, C₁-C₆-alkyl and/or with the group -NR¹²R¹³ ; or for aryl or hetaryl that is optionally substituted in one or more places in the same way or differently with halogen, hydroxy, C₁-C₆₋alkyloxy, aralkyloxy, C₁-C₆-alkyl, halo-C₁-C₆-alkyl, cyano-C₁-C₆-alkyl or with the group =O, OR¹⁴, or R¹⁴,
Y₁ to Y₅ in each case stand for a nitrogen atom or for the group -CY⁶,
Y⁶ stands for cyano, halogen, C₁-C₆-alkyl, C₁-C₆₋alkoxy, amino or hydroxy, wherein at least one nitrogen atom is contained in the ring and at least one cyano radical is contained on the ring,
D stands for a nitrogen atom or for the group C-R³,
E stands for a nitrogen atom or for the group C-R⁴,
F stands for a nitrogen atom or for the group C-R⁵,
G stands for a nitrogen atom or for the group C-R⁶, wherein
R³, R⁴, R⁵ and R⁶ stand for hydrogen, halogen or C₁-C₆-alkoxy, C₁-C₆-alkyl or C₁-C₆-carboxyalkyl that is unsubstituted or that is optionally substituted in one or more places with halogen,
R⁷ stands for hydrogen or C₁-C₆-alkyl, or forms a bridge with up to 3 ring members with Rₐ-R_{f} from Z or to form R¹,
R⁹ stands for hydrogen or C₁-C₆-alkyl,
R¹² and R¹³ stand for hydrogen, C₁-C₆-alkyl or form a ring that can contain another heteroatom,
R¹⁴ stands for the group (CH₂-CH₂-O)ᵤ(CH₂)ᵥ-R¹⁵,
R¹⁵ stands for aryl, hetaryl, C₁₋₆-alkyl, aralkyl, -CH₂CN or for the group NR¹⁶R¹⁷,
R¹⁶ and R¹⁷ stand for hydrogen, C₁-C₆-alkyl, C₁-C₆₋acyl or form a ring that can contain another heteroatom, and
u and v stand for 0-5, as well as isomers, enantiomers and salts thereof.

3. Compounds of general formula I, according to Claims 1 and 2, in which
A stands for the group -N(R⁷)-,
W stands for oxygen,
Z stands for a bond or for branched or unbranched C₁-C₁₂-alkyl,
X stands for C₁-C₆-alkyl,
R¹ stands for aryl or hetaryl that is optionally substituted in one or more places in the same way or differently with halogen, hydroxy, C₁-C₆₋alkyloxy, aralkyloxy, C₁-C₆-alkyl, halo-C₁-C₆-alkyl, cyano-C₁-C₆-alkyl or with the group =O, -OR¹⁴, or R¹⁴,
Y₁ to Y₅ together stand for a pyridyl ring which is substituted with a cyano group and which in addition can be substituted with halogen, C₁-C₆₋alkyl, C₁-C₆-alkoxy, amino, C₁-C₆-carboxyalkylamino or hydroxy,
D stands for a nitrogen atom or for the group C-R³,
E stands for a nitrogen atom or for the group C-R⁴,
F stands for a nitrogen atom or for the group C-R⁵,
G stands for a nitrogen atom or for the group C-R⁶, wherein
R³, R⁴, R⁵ and R⁶ stand for hydrogen, halogen, or C₁-C₆-alkoxy, C₁-C₆-alkyl or C₁-C₆-carboxyalkyl that is unsubstituted or that is optionally substituted in one or more places with halogen,
R⁷ stands for hydrogen or C₁-C₆-alkyl or forms a bridge with up to 3 ring members with Rₐ-R_{f} from Z or to form R¹,
R⁹ stands for hydrogen or C₁-C₆-alkyl,
R¹⁴ stands for the group (CH₂-CH₂-O)ᵤ(CH₂)ᵥ-R¹⁵,
R¹⁵ stands for aryl, hetaryl, C₁₋₆-alkyl, aralkyl, -CH₂CN or for the group NR¹⁶R¹⁷,
R¹⁶ and R¹⁷ stand for hydrogen, C₁-C₆-alkyl, C₁-C₆₋acyl or form a ring that can contain another heteroatom, and
u and v stand for 0-5, as well as isomers, enantiomers and salts thereof.

4. Compounds of general formula I, according to Claims 1 to 3, in which
A stands for the group -N(R⁷)-,
W stands for oxygen,
Z stands for a bond or C₁-C₆-alkyl,
X stands for C₁-C₆-alkyl,
R¹ stands for thiophene, furan, oxazole, thiazole, indazolyl, imidazole, pyrazole, pyridine, pyrimidine, triazine, quinoline, isoquinoline or 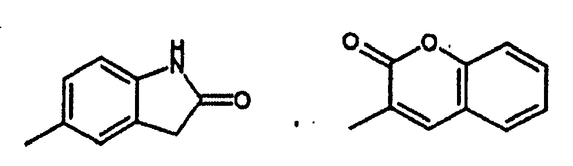 or 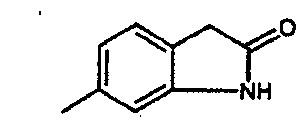 that is optionally substituted in one or more places in the same way or differently with halogen, hydroxy, C₁-C₆-alkyloxy, aralkyloxy, C₁-C₆₋alkyl, halo-C₁-C₆-alkyl, cyano-C₁-C₆-alkyl or with the group =O,
Y₁ to Y₅ together stand for a pyridyl ring, which is substituted with a cyano group and which in addition can be substituted with halogen,
D stands for a nitrogen atom or for the group C-R³,
E stands for the group C-R⁴,
F stands for the group C-R⁵,
G stands for the group C-R⁶, wherein
R³, R⁴, R⁵ and R⁶ stand for hydrogen,
R⁷ stands for hydrogen, and
R⁹ stands for hydrogen, as well as isomers, enantiomers and salts thereof.

5. Compounds of general formula I, according to Claims 1 to 4, in which
A stands for the group -N(R⁷)-,
W stands for oxygen,
Z stands for a bond,
X stands for C₁-C₆-alkyl,
R¹ stands for indazolyl, quinoline, isoquinoline or 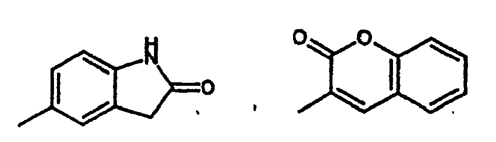 or 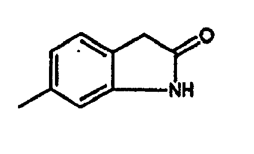 that is optionally substituted in one or more places in the same way or differently with C₁-C₆₋alkyloxy, C₁-C₆-alkyl, cyano-C₁-C₆-alkyl or with the group =O,
Y₁ to Y₅ together stand for a pyridyl ring, which is substituted with a cyano group and which in addition can be substituted with halogen,
D stands for a nitrogen atom or for the group C-R³,
E stands for the group C-R⁴,
F stands for the group C-R⁵,
G stands for the group C-R⁶, wherein
R³, R⁴, R⁵ and R⁶ stand for hydrogen,
R⁷ stands for hydrogen, and
R⁹ stands for hydrogen, as well as isomers, enantiomers and salts thereof.

6. Pharmaceutical agents that contain at least one compound according to Claims 1 to 5.

7. Pharmaceutical agents according to Claim 6 for use in the case of psoriasis, Kaposi's sarcoma, restenosis, such as, e.g., stent-induced restenosis, endometriosis, Crohn's disease, Hodgkin's disease, leukaemia; arthritis, such as rheumatoid arthritis, haemangioma, angiofibroma; eye diseases, such as diabetic retinopathy, neovascular glaucoma; renal diseases, such as glomerulonephritis, diabetic nephropathy, malignant nephrosclerosis, thrombic microangiopathic syndrome, transplant rejections and glomerulopathy; fibrotic diseases, such as cirrhosis of the liver, mesangial cell proliferative diseases, arteriosclerosis, injuries to nerve tissue, inhibition of the reocclusion of vessels after balloon catheter treatment, vascular prosthetics or use of mechanical devices to keep vessels open, such as, e.g., stents, and as immunosuppressive agents, and for supporting scar-free healing, and in senile keratosis and in contact dermatitis.

8. Compounds according to Claims 1 to 5 and pharmaceutical agents, according to Claims 6 and 7, with suitable formulation substances and vehicles.

9. Use of the compounds of formula I, according to Claims 1 to 5, as inhibitors of the tyrosine kinases KDR and FLT.

10. Use of the compounds of general formula I, according to Claims 1 to 5, in the form of a pharmaceutical preparation for enteral, parenteral and oral administration.

11. Use of the compounds according to Claims 1 to 5 in the case of psoriasis, Kaposi's sarcoma, restenosis, such as, e.g., stent-induced restenosis, endometriosis, Crohn's disease, Hodgkin's disease, leukaemia; arthritis, such as rheumatoid arthritis, haemangioma, angiofibroma; eye diseases, such as diabetic retinopathy, neovascular glaucoma; renal diseases, such as glomerulonephritis, diabetic nephropathy, malignant nephrosclerosis, thrombic microangiopathic syndrome, transplant rejections and glomerulopathy; fibrotic diseases, such as cirrhosis of the liver, mesangial cell proliferative diseases, arteriosclerosis, injuries to nerve tissue, and for inhibiting the reocclusion of vessels after balloon catheter treatment, in vascular prosthetics or after mechanical devices are used to keep vessels open, such as, e.g., stents, and as immunosuppressive agents, and for supporting scar-free healing, and in senile keratosis and in contact dermatitis.

12. Use of the compounds of general formula I, according to Claims 1 to 5, as VEGFR kinase 3 inhibitors of lymphangiogenesis.

## Revendications

1. Composés de formule générale I 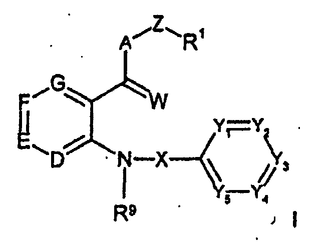 dans laquelle
A représente le groupe -N(R⁷)-,
W représente un atome d'oxygène, un atome de soufre, deux atomes d'hydrogène ou le groupe -N(R⁸)-,
Z représente une liaison, le groupe -N (R¹⁰)- ou =N-, un groupe alkyle en C₁-C₁₂ ramifié ou non ramifié, ou le groupe 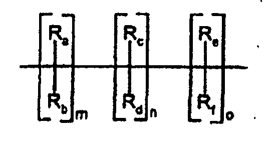
m, n et o représente 0-3,
Rₐ, R_{b}, R_{c}, R_{d}, Rₑ, R_{f} représentent, indépendamment les uns des autres, un atome d'hydrogène ou de fluor, un groupe alkyle en C₁-C₄ ou le groupe -N(R¹¹)- et/ou Rₐ et/ou R_{b} avec R_{c} et/ou R_{d} ou R_{c} avec Rₑ et/ou R_{f} peuvent former une liaison, ou jusqu'à deux des radicaux Rₐ₋R_{f} peuvent former un pont, ayant chaque fois jusqu'à 3 atomes de carbone, avec R¹ ou avec R⁷,
X représente un groupe alkyle en C₁-C₆,
R¹ représente un groupe alkyle en C₁-C₁₂ ou alcényle en C₂-C₁₂ ramifié ou non ramifié, éventuellement une ou plusieurs fois substitué, de façon identique ou différente, par halogène, hydroxy, alkyloxy en C₁-C₆, aralkyloxy, alkyle en C₁-C₆ et/ou par le groupe -NR¹²R¹³; ou représente un groupe cycloalkyle en C₃-C₁₀ ou cycloalcényle en C₃-C₁₀ éventuellement une ou plusieurs fois substitué, de façon identique ou différente, par halogène, hydroxy, alkyloxy en C₁-C₆, alkyle en C₁-C₆ et/ou par le groupe -NR¹²R¹³ ; ou représente un groupe aryle ou hétéroaryle éventuellement une ou plusieurs fois substitué, de façon identique ou différente, par halogène, hydroxy, alkyloxy en C₁-C₆, aralkyloxy, alkyle en C₁-C₆, halogéno-alkyle en C₁-C₆, cyano-alkyle en C₁-C₆ ou par le groupe =O, OR¹⁴ ou R¹⁴,
Y₁ à Y₅ représentent chacun un atome d'azote ou le groupe -CY⁶,
Y⁶ représente un groupe cyano, un atome d'halogène, un groupe alkyle en C₁-C₆, alcoxy en C₁-C₆, amino ou hydroxy, au moins un atome d'azote étant contenu dans le cycle et au moins un radical cyano étant contenu dans le cycle,
D représente un atome d'azote ou le groupe C-R³,
E représente un atome d'azote ou le groupe C-R⁴,
F représente un atome d'azote ou le groupe C-R⁵,
G représente un atome d'azote ou le groupe C-R⁶,
R³, R⁴, R⁵ et R⁶ représentant un atome d'hydrogène ou d'halogène, ou un groupe alcoxy en C₁-C₆, alkyle en C₁-C₆ ou carboxyalkyle en C₁-C₆, non substitué ou éventuellement une ou plusieurs fois substitué par halogène,
R⁷ représente un atome d'hydrogène ou un groupe alkyle en C₁-C₆ ou forme avec Rₐ-R_{f} de Z ou avec R¹ un pont ayant jusqu'à 3 chaînons formant le cycle,
R⁸, R⁹, R¹⁰ et R¹¹ représentent un atome d'hydrogène ou un groupe alkyle en C₁-C₆,
R²² et R¹³ représentent un atome d'hydrogène, un groupe alkyle en C₁-C₆, ou forment un cycle qui peut contenir un autre hétéroatome,
R¹⁴ représente le groupe alkyl (C₁-C₁₅)-R¹⁵, dans lequel le radical alkyle peut être interrompu une ou plusieurs fois par un atome d'oxygène, ou représente le groupe (CH₂-CH₂-O)ᵤ(CH₂)ᵥ-R¹⁵,
R¹⁵ représente un groupe aryle, hétéroaryle, alkyle en C₁-C₆, aralkyle, -CH₂CN ou le groupe NR¹⁶R¹⁷,
R¹⁶ et R¹⁷ représentent un atome d'hydrogène, un groupe alkyle en C₁-C₆, acyle en C₁-C₆ ou forment un cycle qui peut contenir un autre hétéroatome, et
u et v représentent 0 à 5,
ainsi que leurs isomères, énantiomères et sels.

2. Composés de formule générale I, selon la revendication 1, dans lesquels
A représente le groupe -N(R⁷)-,
W représente un atome d'oxygène,
Z représente une liaison ou un groupe alkyle en C₁-C₁₂ ramifié ou non ramifié,
X représente un groupe alkyle en C₁-C₆,
R¹ représente un groupe alkyle en C₁-C₁₂ ou alcényle en C₂-C₁₂ ramifié ou non ramifié, éventuellement une ou plusieurs fois substitué, de façon identique ou différente, par halogène, hydroxy, alkyloxy en C₁-C₆, aralkyloxy, alkyle en C₁-C₆ et/ou par le groupe -NR¹²R¹³; ou représente un groupe cycloalkyle en C₃-C₁₀ ou cycloalcényle en C₃-C₁₀ éventuellement une ou plusieurs fois substitué, de façon identique ou différente, par halogène, hydroxy, alkyloxy en C₂-C₆, alkyle en C₁-C₆ et/ou par le groupe *-*NR¹²R¹³ ; ou représente un groupe aryle ou hétéroaryle éventuellement une ou plusieurs fois substitué, de façon identique ou différente, par halogène, hydroxy, alkyloxy en C₁-C₆, aralkyloxy, alkyle en C₁-C₆, halogéno-alkyle en C₁-C₆, cyano-alkyle en C₁-C₆ ou par le groupe =O, OR¹⁴ ou R¹⁴,
Y₁ à Y₅ représentent chacun un atome d'azote ou le groupe -CY⁶,
Y⁶ représente un groupe cyano, un atome d'halogène, un groupe alkyle en C₁-C₆, alcoxy en C₁-C₆, amino ou hydroxy, au moins un atome d'azote étant contenu dans le cycle et au moins un radical cyano étant contenu dans le cycle,
D représente un atome d'azote ou le groupe C-R³,
E représente un atome d'azote ou le groupe C-R⁴,
F représente un atome d'azote ou le groupe C-R⁵,
G représente un atome d'azote ou le groupe C-R⁶,
R³, R⁴, R⁵ et R⁶ représentant un atome d'hydrogène ou d'halogène, ou un groupe alcoxy en C₁-C₆, alkyle en C₁-C₆ ou carboxyalkyle en C₁-C₆, non substitué ou éventuellement une ou plusieurs fois substitué par halogène,
R⁷ représente un atome d'hydrogène ou un groupe alkyle en C₁-C₆ ou forme avec Rₐ-R_{f} de Z ou avec R¹ un pont ayant jusqu'à 3 chaînons formant le cycle,
R⁹ représente un atome d'hydrogène ou un groupe alkyle en C₁-C₆,
R¹² et R¹³ représentent un atome d'hydrogène, un groupe alkyle en C₁-C₆, ou forment un cycle qui peut contenir un autre hétéroatome,
R¹⁴ représente le groupe (CH₂-CH₂-O)ᵤ(CH₂)ᵥ-R¹⁵,
R¹⁵ représente un groupe aryle, hétéroaryle, alkyle en C₁-C₆, aralkyle, -CH₂CN ou le groupe NR¹⁶R¹⁷,
R¹⁶ et R¹⁷ représentent un atome d'hydrogène, un groupe alkyle en C₁-C₆, acyle en C₁-C₆ ou forment un cycle qui peut contenir un autre hétéroatome, et
u et v représentent 0 à 5,
ainsi que leurs isomères, énantiomères et sels.

3. Composés de formule générale I, selon les revendications 1 et 2, dans lesquels
A représente le groupe -N(R⁷)-,
W représente un atome d'oxygène,
Z représente une liaison ou un groupe alkyle en C₁-C₁₂ ramifié ou non ramifié,
X représente un groupe alkyle en C₁-C₆,
R¹ représente un groupe aryle ou hétéroaryle éventuellement une ou plusieurs fois substitué, de façon identique ou différente, par halogène, hydroxy, alkyloxy en C₁-C₆, aralkyloxy, alkyle en C₁-C₆, halogéno-alkyle en C₁-C₆, cyano-alkyle en C₁-C₆ ou par le groupe =O, OR¹⁴ ou R¹⁴,
Y₁ à Y₅ représentent ensemble un cycle pyridyle qui est substitué par un groupe cyano et qui peut en outre être substitué par halogène, alkyle en C₁-C₆, alcoxy en C₁-C₆, amino, carboxyalkyl-(C₁-C₆) amino ou hydroxy,
D représente un atome d'azote ou le groupe C-R³,
E représente un atome d'azote ou le groupe C-R⁴,
F représente un atome d'azote ou le groupe C-R⁵,
G représente un atome d'azote ou le groupe C-R⁶,
R³, R⁴ R⁵ et R⁶ représentant un atome d'hydrogène ou d'halogène, ou un groupe alcoxy en C₁-C₆, alkyle en C₁-C₆ ou carboxyalkyle en C₁-C₆, non substitué ou éventuellement une ou plusieurs fois substitué par halogène,
R⁷ représente un atome d'hydrogène ou un groupe alkyle en C₁-C₆ ou forme avec Rₐ-R_{f} de Z ou avec R¹ un pont ayant jusqu'à 3 chaînons formant le cycle,
R⁹ représente un atome d'hydrogène ou un groupe alkyle en C₁-C₆,
R¹⁴ représente le groupe (CH₂-CH₂-O)ᵤ(CH₂)ᵥ-R¹⁵,
R¹⁵ représente un groupe aryle, hétéroaryle, alkyle en C₁-C₆, aralkyle, -CH₂CN ou le groupe NR¹⁶R¹⁷,
R¹⁶ et R¹⁷ représentent un atome d'hydrogène, un groupe alkyle en C₁-C₆, acyle en C₁-C₆ ou forment un cycle qui peut contenir un autre hétéroatome, et
u et v représentent 0 à 5,
ainsi que leurs isomères, énantiomères et sels.

4. Composés de formule générale I, selon les revendications 1 à 3, dans lesquels
A représente le groupe -N(R⁷)-,
W représente un atome d'oxygène,
Z représente une liaison ou un groupe alkyle en C₁-C₆,
X représente un groupe alkyle en C₁-C₆,
R¹ représente un groupe thiophène, furanne, oxazole, thiazole, indazolyle, imidazole, pyrazole, pyridine, pyrimidine, triazine, quinoléine, isoquinoléine ou 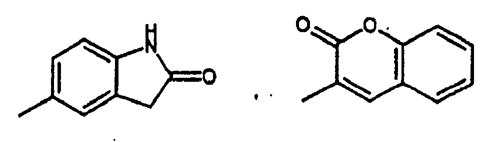 ou 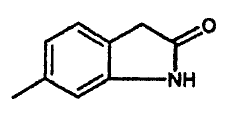 éventuellement une ou plusieurs fois substitué, de façon identique ou différente, par halogène, hydroxy, alkyloxy en C₁-C₆, aralkyloxy, alkyle en C₁-C₆, halogéno-alkyle en C₁-C₆, cyano-alkyle en C₁-C₆ ou par le groupe =0,
Y₁ Y₅ représentent ensemble un cycle pyridyle qui est substitué par un groupe cyano et qui peut en outre être substitué par halogène,
D représente un atome d'azote ou le groupe C-R³,
E représente le groupe C-R⁴,
F représente le groupe C-R⁵,
G représente le groupe C-R⁶,
R³, R⁴, R⁵ et R⁶ représentant un atome d'hydrogène,
R⁷ représente un atome d'hydrogène et
R⁹ représente un atome d'hydrogène,
ainsi que leurs isomères, énantiomères et sels.

5. Composés de formule générale I, selon les revendications 1 à 4, dans lesquels
A représente le groupe -N(R⁷)-,
W représente un atome d'oxygène,
Z représente une liaison,
X représente un groupe alkyle en C₁-C₆,
R¹ représente un groupe indazolyle, quinoléine, isoquinoléine ou 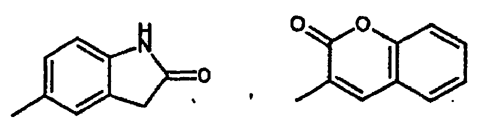 où 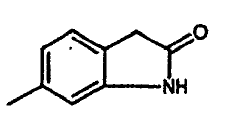 éventuellement une ou plusieurs fois substitué, de façon identique ou différente, par alkyloxy en C₁-C₆, alkyle en C₁-C₆, cyano-alkyle en C₁-C₆ ou par le groupe =O,
Y₁ à Y₅ représentent ensemble un cycle pyridyle qui est substitué par un groupe cyano et qui peut en outre être substitué par halogène,
D représente un atome d'azote ou le groupe C-R³,
E représente le groupe C-R⁴,
F représente le groupe C-R⁵,
G représente le groupe C-R⁶,
R³, R⁴, R⁵ et R⁶ représentant un atome d'hydrogène,
R⁷ représente un atome d'hydrogène et
R⁹ représente un atome d'hydrogène,
ainsi que leurs isomères, énantiomères et sels.

6. Médicament contenant au moins un composé selon les revendications 1 à 5.

7. Médicament selon la revendication 6, pour utilisation dans le psoriasis, le sarcome de Kaposi, la resténose, comme par exemple la resténose induite par stent, l'endométriose, la maladie de Crohn, la maladie de Hodgkin, la leucémie, l'arthrite, telle que la polyarthrite rhumatoïde, l'hémangiome, l'angiofibrome, des maladies des yeux telles que la rétinopathie diabétique, le glaucome néovasculaire, des maladies rénales telles que la glomérulonéphrite, la néphropathie diabétique, la néphrosclérose maligne, les syndromes microangiopathiques thrombotiques, des rejets de greffes et la glomérulopathie, des maladies fibreuses telles que la cirrhose, des maladies prolifératives des cellules mésangiales, l'artériosclérose, des lésions du tissu nerveux, l'inhibition de la ré-occlusion de vaisseaux après traitement par cathéter à ballonnet, pose de prothèses vasculaires ou insertion de dispositifs mécaniques destinés à maintenir ouverts des vaisseaux, comme par exemple des stents, et comme immunosuppresseurs, et pour aider la cicatrisation sans marques, et dans les taches de sénescence et dans la dermatite de contact.

8. Composés selon les revendications 1 à 5 et médicaments selon les revendications 6 et 7, avec des formulations appropriées et des véhicules.

9. Composés de formule I, selon les revendications 1 à 5, pour utilisation en tant qu'inhibiteurs de la tyrosine kinase KDR et FLT.

10. Composés de formule générale I, selon les revendications 1 à 5, pour utilisation sous forme d'une préparation pharmaceutique pour l'administration entérale, parentérale et orale.

11. Composés selon les revendications 1 à 5, pour utilisation dans le psoriasis, le sarcome de Kaposi, la resténose, comme par exemple la resténose induite par stent, l'endométriose, la maladie de Crohn, la maladie de Hodgkin, la leucémie, l'arthrite, telle que la polyarthrite rhumatoïde, l'hémangiome, l'angiofibrome, des maladies des yeux telles que la rétinopathie diabétique, le glaucome néovasculaire, des maladies rénales telles que la glomérulonéphrite, la néphropathie diabétique, la néphrosclérose maligne, les syndromes microangiopathiques thrombotiques, des rejets de greffes et la glomérulopathie, des maladies fibreuses telles que la cirrhose, des maladies prolifératives des cellules mésangiales, l'artériosclérose, des lésions du tissu nerveux et pour l'inhibition de la ré-occlusion de vaisseaux après traitement par cathéter à ballonnet, dans la pose de prothèses vasculaires ou après l'insertion de dispositifs mécaniques destinés à maintenir ouverts des vaisseaux, comme par exemple des stents, et comme immunosuppresseurs, et pour aider la cicatrisation sans marques, et dans les taches de sénescence et dans la dermatite de contact.

12. Composés de formule générale I selon les revendications 1 à 5, pour utilisation en tant qu'inhibiteur de VEGFR kinase 3 de la lymphangiogenèse.
